# EUROPEAN PATENT APPLICATION

(11) **EP 4 403 575 A1**
(43) Date of publication of application: **24.07.2024**
(21) Application number: 22869367.7
(22) Date of filing: 16.09.2022
(51) Int. Cl.: C07K 16/28, A61K 39/395, A61P 37/02, A61P 35/00

(54) **COMPOSITION COMPRISING PD-L1 ANTIGEN-BINDING FRAGMENT AND USE THEREOF**

(30) Priority: 18.09.2021 CN 202111112052
(71) Applicant: Jiangsu Alphamab Biopharmaceuticals Co., Ltd., Jiangsu 215000 (CN); Suzhou Alphamab Co., Ltd., Jiangsu 215125 (CN)
(72) Inventor: XU, Ting, Suzhou, Jiangsu 215000 (CN); GUO, Kangping, Suzhou, Jiangsu 215000 (CN); YUN, Lihong, Suzhou, Jiangsu 215000 (CN); WU, Yuanli, Suzhou, Jiangsu 215000 (CN); HUANG, Yan, Suzhou, Jiangsu 215000 (CN); HU, Hongqin, Suzhou, Jiangsu 215000 (CN)
(74) Representative: Latscha Schöllhorn Partner AG
(86) International application number: PCT/CN2022/119187
(87) International publication number: WO 2023/040999

(57) **Abstract**

The present application relates to a composition comprising an antigen-binding fragment and an excipient. The antigen-binding fragment comprises an immunoglobulin single variable domain capable of specifically binding to PD-L1 and comprising CDR1-3, the CDR3 comprising an amino acid sequence set forth in SEQ ID NO: 47, and the excipient may comprise proline. The composition has good stability and suitable viscosity and can be used for subcutaneous injection.

## Description

### TECHNICAL FIELD

The present application relates to the field of biomedicine and particularly to a composition comprising a PD-L1 antigen-binding fragment and use thereof.

### BACKGROUND

Expression of PD-L1 has been found in several murine and human cancers, including human lung cancer, ovarian cancer, colon cancer, melanoma and various myelomas. Existing results show that the PD-L1 with high expression of tumor cells plays an important role in the immune escape of the tumor by increasing the apoptosis of T cells.

Dosage forms of antibody drugs include an injection formulation, a freeze-dried formulation or the like. For frequently used products and chronic administration, the subcutaneous administration is preferred. This will enable the patient to manage himself, increase the convenience, rapidity and compliance of treatment, save a large amount of expenses for the medical care department, and improve the quality of life of the patient.

Therefore, there is a need to develop high-concentration PD-L1 antibody formulations.

### SUMMARY

The present application provides a composition comprising an antigen-binding fragment specifically binding to PD-L1 and an excipient. The composition comprises at least one of the following benefits: 1) comprising the antigen-binding fragment at a high concentration (e.g., 200 mg/mL); 2) appropriate viscosity; 3) capable of being used for subcutaneous injection and alleviating the pain of a subject; 4) good stability (e.g., accelerated stability and/or long-term stability); 5) avoiding the side effects caused by transfusion; 6) simple components, simple preparation and low cost.

The present application provides a composition comprising an antigen-binding fragment and an excipient, wherein the antigen-binding fragment comprises an immunoglobulin single variable domain capable of specifically binding to PD-L1 and comprising CDR1-3, wherein the CDR3 comprises an amino acid sequence set forth in SEQ ID NO: 47, and wherein the excipient is selected from one or more of the group consisting of: proline, mannitol, sucrose, glycine and L-arginine hydrochloride.

In other embodiments, the excipient may also be selected from one or more of the group consisting of: proline, sucrose, mannitol, sorbitol, glycerol and L-arginine hydrochloride.

In certain embodiments, the excipient is preferably selected from one or more of the group consisting of: proline, sucrose, mannitol, sorbitol, and glycerol; more preferably, the excipient is one or more of proline, sucrose, mannitol and sorbitol.

In certain embodiments, the excipient is L-proline, or the excipient is sucrose, or the excipient is mannitol, or the excipient is sorbitol.

In certain embodiments, the CDR2 comprises an amino acid sequence set forth in SEQ ID NO: 46.

In certain embodiments, the CDR1 comprises an amino acid sequence set forth in SEQ ID NO: 45 or 48.

In certain embodiments, the immunoglobulin single variable domain comprises an amino acid sequence set forth in any one of SEQ ID NOs: 1-6.

In certain embodiments, the PD-L1 is human PD-L1.

In certain embodiments, the antigen-binding fragment comprises an Fc region of an immunoglobulin.

In certain embodiments, the N-terminus of the Fc region of the immunoglobulin is linked directly or indirectly to the C-terminus of the immunoglobulin single variable domain.

In certain embodiments, the N-terminus of the Fc region of the immunoglobulin is linked to the C-terminus of the immunoglobulin single variable domain by a linker.

In certain embodiments, the linker comprises an amino acid sequence set forth in any one of SEQ ID NOs: 42-44.

In certain embodiments, the Fc region of the immunoglobulin comprises an Fc region derived from an immunoglobulin selected from the group consisting of: IgG1, IgG2, IgG3 and IgG4.

In certain embodiments, the Fc region of the immunoglobulin has no ADCC activity; and/or, the Fc region of the immunoglobulin has no CDC activity.

In certain embodiments, the Fc region of the immunoglobulin is derived from a human being. In certain embodiments, the Fc region of the immunoglobulin is derived from human IgG.

In certain embodiments, the Fc region of the immunoglobulin comprises an amino acid sequence set forth in any one of SEQ ID NOs: 7-9.

In certain embodiments, the antigen-binding fragment comprises an amino acid sequence set forth in any one of SEQ ID NOs: 14-31.

In certain embodiments, the antigen-binding fragment is at a concentration of about 1 mg/mL to about 300 mg/mL.

In certain embodiments, the antigen-binding fragment is at a concentration of about 5 mg/mL to about 250 mg/mL.

In certain embodiments, the antigen-binding fragment is at a concentration of about 1 mg/mL to about 220 mg/mL.

In certain embodiments, the antigen-binding fragment is at a concentration of about 20 mg/mL to about 220 mg/mL.

In certain embodiments, the antigen-binding fragment is at a concentration of about 50 mg/mL to about 200 mg/mL.

In certain embodiments, the antigen-binding fragment is at a concentration of about 100 mg/mL to about 280 mg/mL.

In certain embodiments, the antigen-binding fragment is at a concentration of about 150 mg/mL to about 250 mg/mL.

In certain embodiments, the antigen-binding fragment is at a concentration of about 180 mg/mL to about 250 mg/mL.

In certain embodiments, the antigen-binding fragment is at a concentration of about 180 mg/mL to about 220 mg/mL.

In certain embodiments, the excipient is at a concentration of about 50 mM to about 500 mM.

In certain embodiments, the excipient comprises proline at a concentration of about 50 mM to about 390 mM.

In certain embodiments, the excipient comprises proline at a concentration of about 150 mM to about 250 mM.

In certain embodiments, the excipient comprises proline at a concentration of about 150 mM to about 220 mM.

In certain embodiments, the excipient comprises L-proline at a concentration of about 150 mM to about 220 mM.

In certain embodiments, the excipient comprises L-proline at a concentration of about 165 mM to about 275 mM.

In certain embodiments, the excipient comprises mannitol at a concentration of about 50 mM to about 500 mM.

In certain embodiments, the excipient comprises sucrose at a concentration of about 50 mM to about 500 mM.

In certain embodiments, the excipient comprises sucrose at a concentration of about 90 mM to about 500 mM.

In certain embodiments, the excipient comprises glycine at a concentration of about 50 mM to about 500 mM.

In certain embodiments, the excipient comprises glycine at a concentration of about 150 mM to about 250 mM.

In certain embodiments, the excipient comprises L-arginine hydrochloride at a concentration of about 50 mM to about 500 mM.

In certain embodiments, the excipient comprises L-arginine hydrochloride at a concentration of about 150 mM to about 500 mM.

In certain embodiments, the excipient comprises sorbitol at a concentration of about 50 mM to about 500 mM.

In certain embodiments, the excipient comprises sorbitol at a concentration of about 100 mM to about 300 mM.

In certain embodiments, the excipient comprises glycerol at a concentration of 50 mM to about 500 mM.

In certain embodiments, the excipient comprises glycerol at a concentration of about 100 mM to about 300 mM.

In certain embodiments, the composition has a pH of about 5.0 to about 7.5.

In certain embodiments, the composition has a pH of about 5.9 to about 6.9.

In certain embodiments, the composition has a pH of about 6.3 to about 6.5.

In certain embodiments, the composition has a pH of about 6.4.

In certain embodiments, the composition comprises a buffering component, wherein the buffering component is selected from one or more of the group consisting of: sodium acetate-acetic acid, histidine-acetic acid, L-histidine-L-histidine hydrochloride, sodium phosphate and citric acid-sodium hydroxide.

In certain embodiments, the buffering component is selected from one or more of the group consisting of: sodium acetate-acetic acid, L-histidine-L-histidine hydrochloride and citric acid-sodium hydroxide; preferably, the buffering component is sodium acetate-acetic acid or L-histidine-L-histidine hydrochloride.

In certain embodiments, the buffering component is at a concentration of about 5 mM to about 50 mM.

In certain embodiments, the buffering component comprises sodium acetate-acetic acid at a concentration of about 5 mM to about 35 mM.

In certain embodiments, the buffering component comprises sodium acetate-acetic acid at a concentration of about 10 mM to about 30 mM.

In certain embodiments, the buffering component comprises sodium acetate-acetic acid at a concentration of about 20 mM.

In certain embodiments, the buffering component comprises L-histidine-L-histidine hydrochloride at a concentration of about 5 mM to about 35 mM, preferably about 10 mM to about 30 mM, and more preferably 20 mM. In certain embodiments, the composition comprises a surfactant, wherein the surfactant is selected from one or more of the group consisting of: polysorbate 20, polysorbate 80 and poloxamer 188.

In certain embodiments, the surfactant is at a concentration of about 0 mg/mL to about 0.8 mg/mL.

In certain embodiments, the surfactant comprises polysorbate 20 at a concentration of about 0 mg/mL to about 0.4 mg/mL.

In certain embodiments, the surfactant comprises polysorbate 20 at a concentration of about 0.2 mg/mL to about 0.4 mg/mL.

In certain embodiments, the surfactant comprises polysorbate 20 at a concentration of about 0.1 mg/mL to about 0.3 mg/mL.

In certain embodiments, the surfactant comprises polysorbate 20 at a concentration of about 0.2 mg/mL.

In certain embodiments, the surfactant comprises polysorbate 80 at a concentration of about 0.05 mg/mL to about 1 mg/mL, preferably 0.1 mg/mL to 0.5 mg/mL, more preferably 0.1 mg/mL to 0.3 mg/mL, and even more preferably 0.2 mg/mL.

In certain embodiments, the surfactant comprises poloxamer 188 at a concentration of about 0.05 mg/mL to about 5 mg/mL, preferably 0.1 mg/mL to about 3 mg/mL, and more preferably 0.2 mg/mL to about 2 mg/mL.

In certain embodiments, the composition comprises: 1) about 20 mg/mL to about 220 mg/mL of the antigen-binding fragment described herein; 2) about 5 mM to about 35 mM of sodium acetate-acetic acid; 3) about 50 mM to about 390 mM of proline; and 4) about 0 mg/mL to about 0.4 mg/mL of polysorbate 20; and the composition has a pH of about 5.9 to about 6.9.

In certain embodiments, the composition comprises: 1) about 50 mg/mL to about 220 mg/mL of the antigen-binding fragment described herein; 2) about 10 mM to about 30 mM of sodium acetate-acetic acid; 3) about 150 mM to about 250 mM of proline; and 4) about 0.2 mg/mL to about 0.4 mg/mL of polysorbate 20; and the composition has a pH of about 6.3 to about 6.5.

In certain embodiments, the composition comprises: 1) about 150 mg/mL to about 250 mg/mL of the antigen-binding fragment described herein; 2) about 10 mM to about 30 mM of sodium acetate-acetic acid; 3) about 150 mM to about 250 mM of proline; and 4) about 0.2 mg/mL to about 0.4 mg/mL of polysorbate 20; and the composition has a pH of about 6.3 to about 6.5.

In certain embodiments, the composition comprises: 1) about 200 mg/mL of the antigen-binding fragment described herein; 2) about 20 mM of sodium acetate-acetic acid; 3) about 220 mM of proline; and 4) about 0.2 mg/mL of polysorbate 20; and the composition has a pH of about 6.4.

In certain embodiments, the composition comprises: 1) about 100 mg/mL to about 280 mg/mL of the antigen-binding fragment described herein; 2) about 5 mM to about 35 mM of sodium acetate-acetic acid; 3) about 50 mM to about 500 mM of sucrose; and 4) about 0.05 mg/mL to about 0.4 mg/mL of polysorbate 20; and the composition has a pH of about 5.9 to about 6.9.

In certain embodiments, the composition comprises: 1) about 150 mg/mL to about 250 mg/mL of the antigen-binding fragment described herein; 2) about 10 mM to about 30 mM of sodium acetate-acetic acid; 3) about 50 mM to about 300 mM of sucrose; and 4) about 0.1 mg/mL to about 0.3 mg/mL of polysorbate 20; and the composition has a pH of about 6.3 to about 6.5.

In certain embodiments, the composition comprises: 1) about 100 mg/mL to about 280 mg/mL of the antigen-binding fragment described herein; 2) about 5 mM to about 35 mM of sodium acetate-acetic acid; 3) about 50 mM to about 500 mM of mannitol; and 4) about 0.05 mg/mL to about 0.4 mg/mL of polysorbate 20; and the composition has a pH of about 5.9 to about 6.9.

In certain embodiments, the composition comprises: 1) about 150 mg/mL to about 250 mg/mL of the antigen-binding fragment described herein; 2) about 10 mM to about 30 mM of sodium acetate-acetic acid; 3) about 100 mM to about 300 mM of mannitol; and 4) about 0.1 mg/mL to about 0.3 mg/mL of polysorbate 20; and the composition has a pH of about 6.3 to about 6.5.

In certain embodiments, the composition comprises: 1) about 100 mg/mL to about 280 mg/mL of the antigen-binding fragment described herein; 2) about 5 mM to about 35 mM of sodium acetate-acetic acid; 3) about 50 mM to about 500 mM of sorbitol; and 4) about 0.05 mg/mL to about 0.4 mg/mL of polysorbate 20; and the composition has a pH of about 5.9 to about 6.9.

In certain embodiments, the composition comprises: 1) about 150 mg/mL to about 250 mg/mL of the antigen-binding fragment described herein; 2) about 10 mM to about 30 mM of sodium acetate-acetic acid; 3) about 100 mM to about 300 mM of sorbitol; and 4) about 0.1 mg/mL to about 0.3 mg/mL of polysorbate 20; and the composition has a pH of about 6.3 to about 6.5.

In certain embodiments, the composition comprises: 1) about 100 mg/mL to about 280 mg/mL of the antigen-binding fragment described herein; 2) about 5 mM to about 35 mM of sodium acetate-acetic acid; 3) about 50 mM to about 500 mM of glycerol; and 4) about 0.05 mg/mL to about 0.4 mg/mL of polysorbate 20; and the composition has a pH of about 5.9 to about 6.9.

In certain embodiments, the composition comprises: 1) about 150 mg/mL to about 250 mg/mL of the antigen-binding fragment described herein; 2) about 10 mM to about 30 mM of sodium acetate-acetic acid; 3) about 100 mM to about 300 mM of glycerol; and 4) about 0.1 mg/mL to about 0.3 mg/mL of polysorbate 20; and the composition has a pH of about 6.3 to about 6.5.

In certain embodiments, the composition comprises: 1) about 150 mg/mL to about 250 mg/mL of the antigen-binding fragment described herein; 2) about 10 mM to about 30 mM of L-histidine-L-histidine hydrochloride; 3) about 150 mM to about 250 mM of proline; and 4) about 0.2 mg/mL to about 0.4 mg/mL of polysorbate 20; and the composition has a pH of about 6.3 to about 6.5.

In certain embodiments, the composition comprises: 1) about 150 mg/mL to about 250 mg/mL of the antigen-binding fragment described herein; 2) about 10 mM to about 30 mM of L-histidine-L-histidine hydrochloride; 3) about 50 mM to about 300 mM of sucrose; and 4) about 0.1 mg/mL to about 0.3 mg/mL of polysorbate 20; and the composition has a pH of about 6.3 to about 6.5.

In certain embodiments, the composition comprises: 1) about 150 mg/mL to about 250 mg/mL of the antigen-binding fragment described herein; 2) about 10 mM to about 30 mM of L-histidine-L-histidine hydrochloride; 3) about 100 mM to about 300 mM of mannitol; and 4) about 0.1 mg/mL to about 0.3 mg/mL of polysorbate 20; and the composition has a pH of about 6.3 to about 6.5.

In certain embodiments, the composition comprises: 1) about 150 mg/mL to about 250 mg/mL of the antigen-binding fragment described herein; 2) about 10 mM to about 30 mM of L-histidine-L-histidine hydrochloride; 3) about 100 mM to about 300 mM of sorbitol; and 4) about 0.1 mg/mL to about 0.3 mg/mL of polysorbate 20; and the composition has a pH of about 6.3 to about 6.5.

In certain embodiments, the composition comprises: 1) about 150 mg/mL to about 250 mg/mL of the antigen-binding fragment described herein; 2) about 10 mM to about 30 mM of L-histidine-L-histidine hydrochloride; 3) about 100 mM to about 300 mM of glycerol; and 4) about 0.1 mg/mL to about 0.3 mg/mL of polysorbate 20; and the composition has a pH of about 6.3 to about 6.5.

In certain embodiments, the composition is used for injection.

In certain embodiments, the composition is used for subcutaneous injection.

In certain embodiments, the composition is a formulation.

In certain embodiments, the composition is a liquid formulation.

In another aspect, the present application provides a kit comprising the composition described herein and a container holding the composition described herein.

In certain embodiments, the container comprises a glass vial.

In certain embodiments, the composition in the container has a volume of about 0.5 mL to about 5.0 mL.

In certain embodiments, the composition in the container has a volume of about 0.5 mL to about 1.5 mL.

In another aspect, the present application provides use of the composition described herein and/or the kit described herein in the preparation of a medicament for preventing, alleviating and/or treating a tumor.

In certain embodiments, the tumor comprises a PD-L1 positive tumor.

In certain embodiments, the tumor comprises a malignant solid tumor.

In another aspect, the present application provides use of the composition described herein and/or the kit described herein in the preparation of a medicament for preventing, alleviating and/or treating an infectious disease.

In certain embodiments, the infectious disease comprises a disease caused by a viral infection, a bacterial infection, a fungal infection and/or a parasitic infection.

Other aspects and advantages of the present application will be readily apparent to those skilled in the art from the following detailed description. Only exemplary embodiments of the present application are shown and described in the following detailed description. As those skilled in the art will recognize, the content of the present application enables those skilled in the art to make changes to the specific embodiments disclosed without departing from the spirit and scope of the invention involved in the present application. Accordingly, descriptions in the drawings and specification are only illustrative rather than restrictive.

### BRIEF DESCRIPTION OF THE DRAWINGS

Specific features of the invention involved in the present application are set forth in appended claims. Features and advantages of the invention involved in the present application will be better understood by reference to the exemplary embodiments and drawings described in detail below. A brief description of the drawings is as below.
FIG. 1 shows the detection results of Tm values of formulation formulas containing the antigen-binding fragment described herein.
FIG. 2 shows the detection results of aggregate tendency of formulation formulas containing the antigen-binding fragment described herein.
FIG. 3 shows the detection results of SEC-HPLC main peak content of formulation formulas containing the antigen-binding fragment described herein.
FIG. 4 shows the detection results of SEC-HPLC aggregate content of formulation formulas containing the antigen-binding fragment described herein.
FIG. 5 shows the detection results of WCX-HPLC acidic component of formulation formulas containing the antigen-binding fragment described herein.
FIG. 6 shows the detection results of viscosity of formulation formulas containing the antigen-binding fragment described herein.
FIG. 7 shows the detection results of binding activity of formulation formulas containing the antigen-binding fragment described herein.
FIG. 8 shows the detection results of blocking activity of formulation formulas containing the antigen-binding fragment described herein.
FIG. 9 shows the detection results of SEC-HPLC main peak content of formulation formulas containing the antigen-binding fragment described herein.
FIG. 10 shows the detection results of SEC-HPLC aggregate content of formulation formulas of the antigen-binding fragment described herein.
FIG. 11 shows the detection results of WCX-HPLC acidic component of formulation formulas of the antigen-binding fragment described herein.

### DETAILED DESCRIPTION

The embodiments of the present invention are described below with reference to specific examples, and other advantages and effects of the present invention will be readily apparent to those skilled in the art from the disclosure of the present specification.

### Definitions of Terms

In the present application, the term "antigen-binding fragment" generally refers to one or more portions of an antibody that retain the ability to specifically interact with an epitope of an antigen (e.g., by binding, steric hindrance, or the like). The antigen binding function of an antibody can also be achieved by: a heavy chain comprising a fragment of Fv, ScFv, dsFv, Fab, Fab' or F(ab')₂, or a light chain comprising a fragment of Fv, scFv, dsFv, Fab, Fab' or F(ab')₂. (1) Fab fragments, i.e., monovalent fragments consisting of VL, VH, CL and CH domains; (2) F(ab')₂ fragments, bivalent fragments comprising two Fab fragments linked by a disulfide bond at the hinge region; (3) Fd fragments consisting of VH and CH domains; (4) Fv fragments consisting of VL and VH domains of a single arm of an antibody; (5) dAb fragments consisting of VH domains (Ward et al., (1989) Nature 341:544-546); (6) isolated complementarity determining regions (CDRs), and (7) combinations of two or more isolated CDRs which may optionally be linked by a linker. For example, the monovalent single-chain molecule Fv (scFv) formed by pairing of VL and VH may also be included (see Bird et al., (1988) Science 242: 423-426; and Huston et al., (1988) Proc.Natl.Acad.Sci. 85: 5879-5883). For example, antibody VHH that lacks the light chain of an antibody and has only heavy chain variable regions may also be included (see, e.g., Kang Xiaozhen et al., Chinese Journal of Biotechnology. 2018, 34(12): 1974-1984). The "antigen-binding portion" may also include an immunoglobulin fusion protein comprising a binding domain selected from: (1) a binding domain polypeptide fused to an immunoglobulin hinge region polypeptide; (2) an immunoglobulin heavy chain CH2 constant region fused to the hinge region; and (3) an immunoglobulin heavy chain CH3 constant region fused to the CH2 constant region.

In the present application, the term "excipient" generally refers to any material chosen as desired for any type or product form desired for the composition. For example, the product form may include liquid, granules, powder, paste, spray, tablets or gel. The excipient may not affect the form of presence or stability of the antigen-binding fragment or any biological activity of the antigen-binding fragment (e.g., specific binding to the corresponding antigen). In the present application, the excipient may act as a stabilizer to improve the stability of the composition and/or the stability of the antigen-binding fragment in the composition.

In the present application, the term "PD-L1" generally refers to programmed cell death protein 1 ligand 1, which may also be referred to as CD274 or B7H1. In the present application, the PD-L1 may be PD-L1 of mammalian origin. For example, the PD-L1 may be of primate origin or rodent origin (e.g., mouse or rat). Human PD-L1 may have the amino acid sequence shown in NCBI accession No. NP_054862.1. The PD-L1 may include native PD-L1 and may also include variants, isoforms, species homologs of PD-L1 and analogs having at least one common epitope with PD-L1.

In the present application, the term "immunoglobulin variable domain" generally refers to a polypeptide molecule consisting of four "framework regions", i.e., "framework region 1" or "FR1", "framework region 2" or "FR2", "framework region 3" or "FR3" and "framework region 4" or "FR4", and three "complementarity determining regions" or "CDRs", i.e., "complementarity determining region 1" or "CDR1", "complementarity determining region 2" or "CDR2" and "complementarity determining region 3" or "CDR3". The immunoglobulin variable domain may have the tertiary structure of a protein. In the present application, the immunoglobulin variable domain may have the function of specifically binding to an antigen (e.g., PD-L1). The immunoglobulin variable domain may comprise the structure of FR1-CDR1-FR2-CDR2-FR3-CDR3-FR4.

In the present application, the term "immunoglobulin single variable domain" generally refers to the immunoglobulin variable domain having a function of specifically binding to an antigen (e.g., PD-L1) independently. For example, the immunoglobulin single variable domain can specifically bind to an antigen or an epitope thereof without pairing with other immunoglobulin variable domains. For example, the immunoglobulin single variable domain may be a heavy chain variable region VH and/or a light chain variable region VL. In the present application, the immunoglobulin single variable domain may be a VHH. In the present application, the VHH may also be referred to as a heavy chain antibody, a nano antibody or a single-domain antibody. CDRs of the VHH may be divided according to the Kabat method (see "Sequence of proteins of immunological interest", US Public Health Services, NIH Bethesda, MD). For example, in the amino acid sequence of VHH, amino acids at positions 1-30 are FR1, amino acids at positions 31-35 are CDR1, amino acids at positions 36-49 are FR2, amino acids at positions 50-65 are CDR2, amino acids at positions 66-94 are FR3, amino acids at positions 95-102 are CDR3, and amino acids at positions 103-113 are FR4. However, it should be noted that the total number of amino acid residues in each CDR may vary and may not correspond to the total number of amino acid residues indicated by the Kabat numbering (that is, one or more positions according to the Kabat numbering may not be occupied in the actual sequence, or the actual sequence may contain more amino acid residues than the number allowed by the Kabat numbering), and therefore the numbering according to Kabat may or may not correspond to the actual numbering of amino acid residues in the actual sequence.

In the present application, the term "composition" generally refers to a mixture comprising the antigen-binding fragment described herein. The composition may comprise at least one pharmaceutically acceptable component, such as an excipient described herein.

In the present application, the term "formulation" generally refers to a product comprising the antigen-binding fragment described herein in a predetermined amount or ratio and any product that is produced by directly or indirectly incorporating a predetermined amount of the antigen-binding fragment described herein into a combination. The formulations described herein may include pharmaceutical formulations, i.e., a product comprising the antigen-binding fragment described herein and the excipient and any product resulting, directly or indirectly, from the combination, complexation or aggregation of any two or more components. The formulation may be in liquid form.

In the present application, the term "buffering component" generally refers to an agent having the function of providing a buffering effect (e.g., against pH change). For example, the buffering component can adjust for changes in pH due to the addition and/or release of acidic or basic substances. For example, the buffering component may include weak acids and conjugate bases thereof, and it may also include weak bases and conjugate acids thereof.

In the present application, the term "surfactant" generally refers to an agent that can protect a protein (e.g., the antigen-binding fragment described herein) from air/solution interface-induced stress or solution/surface-induced stress to reduce aggregation of the protein or minimize the formation of particles in the composition. The surfactant may comprise both hydrophobic and hydrophilic groups. The surfactant may comprise a mixture or combination of one or more surfactants. The surfactant may comprise a nonionic surfactant.

In the present application, the term "PD-L1 positive" generally means that the proportion of PD-L1 expressing cells in a test tissue sample comprising tumor cells and tumor-infiltrating inflammatory cells is higher than the proportion of cells corresponding to a level rating the sample as expressing cell surface PD-L1. For example, the proportion of cells may be determined by immunohistochemistry (IHC). In the present application, the PD-L1 positive may be that at least about 0.01%, at least about 0.5%, at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, or at least about 30% of the cells in total in a sample express PD-L1.

In the present application, the term "tumor" generally refers to damage caused by abnormal growth of cells. For example, the tumor may form a separate tissue mass by rapid, uncontrolled cell proliferation. The tissue mass may be benign, pre-malignant or malignant. The tumor may be considered malignant when it has the structure and/or function that allow it to grow unlimitedly and/or attack normal tissues. The malignant tumor may be used interchangeably with cancer.

In the present application, the term "malignant solid tumor" generally refers to any type of tumor with a certain morphology that has the potential to metastasize.

In the present application, the term "infectious disease" generally refers to local tissue and systemic inflammatory reactions caused by invasion of human bodies by pathogens, such as viruses, bacteria, fungi, parasites and the like, and specific toxins.

In the present application, the term "about" generally refers to the usual error range for the corresponding value. Reference herein to "about" for a value or a parameter includes (and describes) the schemes for that value or parameter itself. If there is a doubt or if there is no acceptable commonly understood meaning for the error range for a particular value or parameter in the art, "about" means ±5% of that value or parameter.

### Detailed Description of the Invention

In one aspect, the present application provides a composition, which may comprise an antigen-binding fragment and an excipient, wherein the antigen-binding fragment may comprise an immunoglobulin single variable domain capable of specifically binding to PD-L1, the immunoglobulin single variable domain may comprise an amino acid sequence set forth in any one of SEQ ID NOs: 1-6, and the excipient is selected from one or more of the group consisting of: proline, mannitol, sucrose, glycine and L-arginine hydrochloride.

### Antigen-binding fragment

In the present application, the antigen-binding fragment can specifically bind to PD-L1. For example, the PD-L1 may be human PD-L1.

In the present application, the antigen-binding fragment may comprise an Fc region of an immunoglobulin. For example, the antigen-binding fragment may consist of an amino acid sequence of the formula A-L-B, wherein A represents an immunoglobulin single variable domain, L represents an amino acid linker or is absent, and B represents a human immunoglobulin Fc region.

For example, the N-terminus of the Fc region of the immunoglobulin may be linked directly or indirectly to the C-terminus of the immunoglobulin single variable domain.

For example, the N-terminus of the Fc region of the immunoglobulin may be linked to the C-terminus of the immunoglobulin single variable domain by a linker.

In the present application, the immunoglobulin single variable domain may comprise antigen complementarity determining region CDR1-3. For example, the CDR1 may comprise an amino acid sequence set forth in SEQ ID NO: 45 or 48. For example, the CDR2 may comprise an amino acid sequence set forth in SEQ ID NO: 46. For example, the CDR3 may comprise an amino acid sequence set forth in SEQ ID NO: 47. For example, the CDR1 may comprise an amino acid sequence set forth in SEQ ID NO: 45; the CDR2 may comprise an amino acid sequence set forth in SEQ ID NO: 46; and the CDR3 may comprise an amino acid sequence set forth in SEQ ID NO: 47. For example, the CDR1 may comprise an amino acid sequence set forth in SEQ ID NO: 48; the CDR2 may comprise an amino acid sequence set forth in SEQ ID NO: 46; and the CDR3 may comprise an amino acid sequence set forth in SEQ ID NO: 47.

In the present application, the linker may have a length of about 1 to about 20 amino acid residues. For example, the linker may have no secondary or higher structure. For example, the linker may be a flexible linker. For example, the linker may be GGGGS, GS or GAP. For example, the linker may comprise an amino acid sequence set forth in any one of SEQ ID NOs: 42-44.

For example, the Fc region of the immunoglobulin may comprise an Fc region derived from an immunoglobulin selected from the group consisting of: IgG1, IgG2, IgG3 and IgG4. The constant region of human IgG1, IgG2, IgG3 or IgG4 may have amino acid sequences shown in entries P01857, P01859, P01860 and P01861, respectively, in the protein database in www.uniprot.org.

For example, the Fc region of the immunoglobulin may be derived from a human being. For example, the Fc region of the immunoglobulin may be derived from human IgG. For example, the Fc region of the immunoglobulin may be derived from human IgG1.

In the present application, the Fc region of the immunoglobulin may not have ADCC activity; and/or, the Fc region of the immunoglobulin may not have CDC activity. For example, the Fc region of the immunoglobulin may lose ADCC activity and/or CDC activity due to mutation. For example, the Fc region of the immunoglobulin may comprise an amino acid sequence set forth in any one of SEQ ID NOs: 7-9.

In the present application, the antigen-binding fragment may have a homodimer. The homodimer may be formed by the interaction of the Fc regions of the immunoglobulin.

In the present application, the antigen-binding fragment may specifically bind to PD-L1 with a *K_{D}* value of less than 1×10⁻⁷ M, less than 1×10⁻⁸ M, less than 1×10⁻⁹ M, less than 1×10⁻¹⁰ M or less than 1×10⁻¹¹ M.

In the present application, the antigen-binding fragment can specifically bind to human PD-L1 and block the interaction of PD-L1 and PD-1. In the present application, the antigen-binding fragment can specifically bind to human PD-L1 and block the interaction of PD-L1 and CD80. In the present application, the antigen-binding fragment may inhibit the growth of the tumor by at least about 10%, at least about 20%, at least about 30%, at least about 40%, at least about 50%, at least about 60%, at least about 70%, at least about 80% or more.

In the present application, the antigen-binding fragment may be resistant to alkali treatment and/or oxidation treatment. For example, the antigen-binding fragment still retains the ability to specifically bind to PD-L1 after at least 8 hours of treatment with a strong alkali (e.g., ammonium bicarbonate). As another example, the antigen-binding fragment still retains the ability to specifically bind to PD-L1 after at least 2 hours of treatment with an oxidizing agent (e.g., 1% hydrogen peroxide).

In the present application, the antigen-binding fragment may comprise an amino acid sequence set forth in any one of SEQ ID NOs: 14-31.

In the present application, the antigen-binding fragment may comprise functional variants of the antigen-binding fragment and fragments thereof, derivatives of the antigen-binding fragment and fragments thereof, analogues of the antigen-binding fragment and fragments thereof and/or homologues of the antigen-binding fragment and fragments thereof.

The functional variant may be a polypeptide having substantially the same amino acid sequence as a naturally occurring sequence or encoded by substantially the same nucleotide sequence as the naturally occurring sequence and capable of having one or more of the activities of the naturally occurring sequence. For example, the functional variant may be a sequence obtained by modifying the residues in an amino acid sequence set forth in any one of SEQ ID NOs: 14-31 while retaining at least one endogenous function (e.g., may specifically bind to human PD-L1). For example, the modification may comprise addition, deletion, substitution, modification, replacement and/or variation of at least one amino acid residue.

The derivative may comprise a sequence obtained by performing any substitution, variation, modification, replacement, deletion and/or addition of one (or more) amino acid residues of an amino acid sequence set forth in any one of SEQ ID NOs: 14-31 while retaining at least one endogenous function.

The homologue may be an amino acid sequence having at least 80%, 85%, 90%, 99.1%, 99.2%, 99.3%, 99.4%, 99.5%, 99.6%, 99.7%, 99.8% or 99.9% identity to the amino acid sequence set forth in any one of SEQ ID NOs: 14-31. To determine the sequence identity, sequence alignments may be performed by various means known to those skilled in the art, e.g., using software like BLAST, BLAST-2, ALIGN, NEEDLE, Megalign (DNASTAR) and the like. Those skilled in the art can determine appropriate parameters for alignment, including any algorithms required to achieve optimal alignment over the full length of the sequences being compared. The analogue may comprise a simulant of a polypeptide that retains at least one endogenous function of the antigen-binding fragment described herein.

The antigen-binding fragment described herein may have deletions, insertions or substitutions of amino acid residues, and the amino acid residues undergo silent changes and result in a functionally equivalent protein. Intentional amino acid substitutions may be made on the basis of similarity in polarity, charge, solubility, hydrophobicity, hydrophilicity, and/or the amphipathic nature of the residues as long as the endogenous function is retained.

In the composition described herein, the antigen-binding fragment may be at a concentration of about 1 mg/mL to about 220 mg/mL. For example, the antigen-binding fragment may be at a concentration of about 20 mg/mL to about 220 mg/mL. For example, the antigen-binding fragment may be at a concentration of about 30 mg/mL to about 220 mg/mL, about 40 mg/mL to about 220 mg/mL, about 50 mg/mL to about 220 mg/mL, about 30 mg/mL to about 200 mg/mL, about 40 mg/mL to about 200 mg/mL or about 50 mg/mL to about 200 mg/mL. For example, the antigen-binding fragment may be at a concentration of about 1 mg/mL to about 300 mg/mL. For example, the antigen-binding fragment may specifically be at a concentration of about 5 mg/mL, 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, 200 mg/mL, 210 mg/mL, 220 mg/mL, 230 mg/mL, 240 mg/mL, 250 mg/mL, 260 mg/mL, 270 mg/mL, 280 mg/mL, 290 mg/mL or 300 mg/mL. For example, the antigen-binding fragment may be at a concentration of about 5 mg/mL to about 250 mg/mL, about 10 mg/mL to about 290 mg/mL, about 20 mg/mL to about 290 mg/mL, about 30 mg/mL to about 290 mg/mL, about 50 mg/mL to about 290 mg/mL, about 60 mg/mL to about 280 mg/mL, about 70 mg/mL to about 280 mg/mL, about 80 mg/mL to about 280 mg/mL, about 100 mg/mL to about 280 mg/mL, about 110 mg/mL to about 250 mg/mL, about 130 mg/mL to about 250 mg/mL, about 150 mg/mL to about 250 mg/mL, about 160 mg/mL to about 250 mg/mL, about 170 mg/mL to about 250 mg/mL, about 180 mg/mL to about 250 mg/mL, about 120 mg/mL to about 220 mg/mL, about 140 mg/mL to about 220 mg/mL, about 150 mg/mL to about 220 mg/mL, about 175 mg/mL to about 220 mg/mL, or about 190 mg/mL to about 220 mg/mL.

For example, the antigen-binding fragment may be at a concentration of about 50 mg/mL to about 200 mg/mL. For example, the antigen-binding fragment may be at a concentration of about 50 mg/mL; for example, the antigen-binding fragment may be at a concentration of about 200 mg/mL.

### Excipient and other ingredients

For example, the excipient may be at a concentration of about 50 mM to about 500 mM. For example, the excipient may be at a concentration of about 50 mM to about 500 mM, about 100 mM to about 500 mM, about 150 mM to about 500 mM, about 160 mM to about 500 mM, about 165 mM to about 500 mM, about 170 mM to about 500 mM, about 100 mM to about 300 mM, about 150 mM to about 300 mM, about 160 mM to about 300 mM, about 165 mM to about 300 mM, about 170 mM to about 300 mM, about 100 mM to about 275 mM, about 150 mM to about 275 mM, about 160 mM to about 275 mM, about 165 mM to about 275 mM, about 170 mM to about 275 mM, about 100 mM to about 220 mM, about 150 mM to about 220 mM, about 160 mM to about 220 mM, about 165 mM to about 220 mM, or about 170 mM to about 220 mM.

For example, the excipient may comprise proline, which may be at a concentration of about 50 mM to about 500 mM. For example, the proline may be at a concentration of about 50 mM to about 500 mM, about 100 mM to about 500 mM, about 150 mM to about 500 mM, about 160 mM to about 500 mM, about 165 mM to about 500 mM, about 170 mM to about 500 mM, 50 mM to about 390 mM, about 100 mM to about 390 mM, about 150 mM to about 390 mM, about 160 mM to about 390 mM, about 165 mM to about 390 mM, about 170 mM to about 390 mM, about 100 mM to about 300 mM, about 150 mM to about 300 mM, about 160 mM to about 300 mM, about 165 mM to about 300 mM, about 170 mM to about 300 mM, about 100 mM to about 275 mM, about 150 mM to about 275 mM, about 160 mM to about 275 mM, about 165 mM to about 275 mM, about 170 mM to about 275 mM, about 100 mM to about 250 mM, about 150 mM to about 250 mM, about 160 mM to about 250 mM, about 165 mM to about 250 mM, about 170 mM to about 250 mM, about 100 mM to about 220 mM, about 150 mM to about 220 mM, about 160 mM to about 220 mM, about 165 mM to about 220 mM, or about 170 mM to about 220 mM. For example, the excipient may comprise proline, which may be at a concentration of about 165 mM to about 275 mM. For example, the excipient may comprise proline, which may be at a concentration of about 150 mM to about 250 mM. For example, the excipient may comprise proline, which may be at a concentration of about 220 mM. For example, the excipient may comprise L-proline, which may be at a concentration of about 50 mM to about 500 mM. For example, the L-proline may be at a concentration of about 50 mM to about 500 mM, about 100 mM to about 500 mM, about 150 mM to about 500 mM, about 160 mM to about 500 mM, about 165 mM to about 500 mM, about 170 mM to about 500 mM, 50 mM to about 390 mM, about 100 mM to about 390 mM, about 150 mM to about 390 mM, about 160 mM to about 390 mM, about 165 mM to about 390 mM, about 170 mM to about 390 mM, about 100 mM to about 300 mM, about 150 mM to about 300 mM, about 160 mM to about 300 mM, about 165 mM to about 300 mM, about 170 mM to about 300 mM, about 100 mM to about 275 mM, about 150 mM to about 275 mM, about 160 mM to about 275 mM, about 165 mM to about 275 mM, about 170 mM to about 275 mM, about 100 mM to about 250 mM, about 150 mM to about 250 mM, about 160 mM to about 250 mM, about 165 mM to about 250 mM, about 170 mM to about 250 mM, about 100 mM to about 220 mM, about 150 mM to about 220 mM, about 160 mM to about 220 mM, about 165 mM to about 220 mM, or about 170 mM to about 220 mM. For example, the excipient may comprise L-proline, which may be at a concentration of about 165 mM to about 275 mM. For example, the excipient may comprise L-proline, which may be at a concentration of about 150 mM to about 250 mM. For example, the excipient may comprise L-proline, which may be at a concentration of about 220 mM.

For example, the excipient may comprise mannitol, which may be at a concentration of about 50 mM to about 500 mM. For example, the mannitol may be at a concentration of about 50 mM to about 500 mM, about 100 mM to about 500 mM, about 150 mM to about 500 mM, about 160 mM to about 500 mM, about 165 mM to about 500 mM, about 170 mM to about 500 mM, 50 mM to about 390 mM, about 100 mM to about 390 mM, about 150 mM to about 390 mM, about 160 mM to about 390 mM, about 165 mM to about 390 mM, about 170 mM to about 390 mM, about 100 mM to about 300 mM, about 150 mM to about 300 mM, about 160 mM to about 300 mM, about 165 mM to about 300 mM, about 170 mM to about 300 mM, about 100 mM to about 275 mM, about 150 mM to about 275 mM, about 160 mM to about 275 mM, about 165 mM to about 275 mM, about 170 mM to about 275 mM, about 100 mM to about 250 mM, about 150 mM to about 250 mM, about 160 mM to about 250 mM, about 165 mM to about 250 mM, about 170 mM to about 250 mM, about 100 mM to about 220 mM, about 150 mM to about 220 mM, about 160 mM to about 220 mM, about 165 mM to about 220 mM, or about 170 mM to about 220 mM.

For example, the excipient may comprise sucrose, which may be at a concentration of about 50 mM to about 500 mM. For example, the sucrose may be at a concentration of about 50 mM to about 500 mM, about 90 mM to about 500 mM, about 100 mM to about 500 mM, about 150 mM to about 500 mM, about 160 mM to about 500 mM, about 165 mM to about 500 mM, about 170 mM to about 500 mM, 50 mM to about 390 mM, about 100 mM to about 390 mM, about 150 mM to about 390 mM, about 160 mM to about 390 mM, about 165 mM to about 390 mM, about 170 mM to about 390 mM, about 100 mM to about 300 mM, about 150 mM to about 300 mM, about 160 mM to about 300 mM, about 165 mM to about 300 mM, about 170 mM to about 300 mM, about 100 mM to about 275 mM, about 150 mM to about 275 mM, about 160 mM to about 275 mM, about 165 mM to about 275 mM, about 170 mM to about 275 mM, about 100 mM to about 250 mM, about 150 mM to about 250 mM, about 160 mM to about 250 mM, about 165 mM to about 250 mM, about 170 mM to about 250 mM, about 100 mM to about 220 mM, about 150 mM to about 220 mM, about 160 mM to about 220 mM, about 165 mM to about 220 mM, about 170 mM to about 220 mM, about 30 mM to about 300 mM, about 40 mM to about 300 mM, about 50 mM to 300 mM, about 60 mM to 300 mM, about 80 mM to 300 mM, about 30 mM to about 275 mM, about 40 mM to about 275 mM, about 50 mM to about 275 mM, about 30 mM to about 250 mM, about 40 mM to about 250 mM, about 50 mM to about 250 mM, about 30 mM to about 220 mM, about 40 mM to about 220 mM, or about 50 mM to about 220 mM.

For example, the excipient may comprise glycine, which may be at a concentration of about 50 mM to about 500 mM. For example, the glycine may be at a concentration of about 50 mM to about 500 mM, about 100 mM to about 500 mM, about 150 mM to about 500 mM, about 160 mM to about 500 mM, about 165 mM to about 500 mM, about 170 mM to about 500 mM, 50 mM to about 390 mM, about 100 mM to about 390 mM, about 150 mM to about 390 mM, about 160 mM to about 390 mM, about 165 mM to about 390 mM, about 170 mM to about 390 mM, about 100 mM to about 300 mM, about 150 mM to about 300 mM, about 160 mM to about 300 mM, about 165 mM to about 300 mM, about 170 mM to about 300 mM, about 100 mM to about 275 mM, about 150 mM to about 275 mM, about 160 mM to about 275 mM, about 165 mM to about 275 mM, about 170 mM to about 275 mM, about 100 mM to about 250 mM, about 150 mM to about 250 mM, about 160 mM to about 250 mM, about 165 mM to about 250 mM, about 170 mM to about 250 mM, about 100 mM to about 220 mM, about 150 mM to about 220 mM, about 160 mM to about 220 mM, about 165 mM to about 220 mM, or about 170 mM to about 220 mM.

For example, the excipient may comprise L-arginine hydrochloride, which may be at a concentration of about 50 mM to about 500 mM. For example, the L-arginine hydrochloride may be at a concentration of about 50 mM to about 500 mM, about 100 mM to about 500 mM, about 150 mM to about 500 mM, about 160 mM to about 500 mM, about 165 mM to about 500 mM, about 170 mM to about 500 mM, 50 mM to about 390 mM, about 100 mM to about 390 mM, about 150 mM to about 390 mM, about 160 mM to about 390 mM, about 165 mM to about 390 mM, about 170 mM to about 390 mM, about 100 mM to about 300 mM, about 150 mM to about 300 mM, about 160 mM to about 300 mM, about 165 mM to about 300 mM, about 170 mM to about 300 mM, about 100 mM to about 275 mM, about 150 mM to about 275 mM, about 160 mM to about 275 mM, about 165 mM to about 275 mM, about 170 mM to about 275 mM, about 100 mM to about 250 mM, about 150 mM to about 250 mM, about 160 mM to about 250 mM, about 165 mM to about 250 mM, about 170 mM to about 250 mM, about 100 mM to about 220 mM, about 150 mM to about 220 mM, about 160 mM to about 220 mM, about 165 mM to about 220 mM, or about 170 mM to about 220 mM.

For example, the excipient may comprise sorbitol, which may be at a concentration of about 50 mM to about 500 mM. For example, the sorbitol may be at a concentration of about 100 mM to about 500 mM, about 130 mM to about 500 mM, about 150 mM to about 500 mM, about 200 mM to about 500 mM, about 80 mM to about 400 mM, about 100 mM to about 400 mM, about 150 mM to about 400 mM, about 180 mM to about 400 mM, about 100 mM to about 350 mM, about 130 mM to about 350 mM, about 150 mM to about 350 mM, about 170 mM to about 350 mM, about 190 mM to about 350 mM, about 75 mM to about 300 mM, about 100 mM to about 300 mM, about 120 mM to about 300 mM, about 150 mM to about 300 mM, about 170 mM to about 300 mM, about 180 mM to about 300 mM, about 200 mM to about 300 mM, about 100 mM to about 275 mM, about 120 mM to about 275 mM, about 150 mM to about 275 mM, about 180 mM to about 275 mM, about 100 mM to about 250 mM, about 130 mM to about 250 mM, about 150 mM to about 250 mM, about 160 mM to about 250 mM, about 170 mM to about 250 mM, about 180 mM to about 250 mM, about 100 mM to about 220 mM, about 120 mM to about 220 mM, about 140 mM to about 220 mM, about 160 mM to about 220 mM, or about 180 mM to about 220 mM.

For example, the excipient may comprise glycerol, which may be at a concentration of about 50 mM to about 500 mM. For example, the sorbitol may be at a concentration of about 100 mM to about 500 mM, about 130 mM to about 500 mM, about 150 mM to about 500 mM, about 200 mM to about 500 mM, about 80 mM to about 400 mM, about 100 mM to about 400 mM, about 150 mM to about 400 mM, about 180 mM to about 400 mM, about 100 mM to about 350 mM, about 130 mM to about 350 mM, about 150 mM to about 350 mM, about 170 mM to about 350 mM, about 190 mM to about 350 mM, about 75 mM to about 300 mM, about 100 mM to about 300 mM, about 120 mM to about 300 mM, about 150 mM to about 300 mM, about 170 mM to about 300 mM, about 180 mM to about 300 mM, about 200 mM to about 300 mM, about 100 mM to about 275 mM, about 120 mM to about 275 mM, about 150 mM to about 275 mM, about 180 mM to about 275 mM, about 100 mM to about 250 mM, about 130 mM to about 250 mM, about 150 mM to about 250 mM, about 160 mM to about 250 mM, about 170 mM to about 250 mM, about 180 mM to about 250 mM, about 100 mM to about 220 mM, about 120 mM to about 220 mM, about 140 mM to about 220 mM, about 160 mM to about 220 mM, or about 180 mM to about 220 mM.

In the present application, the excipient may comprise a stabilizer. The stabilizer may be a component that helps maintain the structural integrity of the composition. For example, the stabilizer may help maintain the structural integrity of the composition during freezing, lyophilization and/or storage. For example, the stabilizer may act as an osmotic agent that mitigates denaturation of proteins (e.g., the antigen-binding fragment described herein).

The excipient described herein may further comprise an antioxidant, which may be selected from one or more of the group consisting of methionine, phenol, cresol, ascorbic acid, glutathione, sodium thiosulfate, citric acid and nicotinamide. For example, the excipient described herein may further comprise L-methionine at a concentration of about 0.1 mM to about 100 mM, about 0.5 mM to about 100 mM, about 1 mM to about 100 mM, about 2 mM to about 100 mM, about 5 mM to about 100 mM, about 0.2 mM to about 50 mM, about 1 mM to about 50 mM, about 3 mM to about 50 mM, about 4 mM to about 50 mM, about 5 mM to about 50 mM, about 0.1 mM to about 30 mM, about 0.3 mM to about 30 mM, about 0.6 mM to about 30 mM, about 1 mM to about 30 mM, about 2 mM to about 30 mM, about 5 mM to about 30 mM, about 0.1 mM to about 20 mM, about 0.5 mM to about 20 mM, about 1 mM to about 20 mM, about 2 mM to about 20 mM, about 0.1 mM to about 10 mM, about 0.2 mM to about 10 mM, about 0.5 mM to about 10 mM, about 0.8 mM to about 10 mM, about 1 mM to about 10 mM, about 1.5 mM to about 10 mM, about 2 mM to about 10 mM, about 2.5 mM to about 10 mM, about 3 mM to about 10 mM, about 4 mM to about 10 mM, or about 5 mM to about 10 mM.

The excipient described herein may further comprise a chelating agent, which may be disodium EDTA at a concentration of about 0.1 mM to about 10 mM, about 0.5 mM to about 10 mM, about 1 mM to about 10 mM, about 0.1 mM to about 5 mM, about 0.2 mM to about 5 mM, about 0.3 mM to about 5 mM, about 0.4 mM to about 5 mM, about 0.5 mM to about 5 mM, about 0.1 mM to about 3 mM, about 0.5 mM to about 3 mM, about 0.6 mM to about 3 mM, about 0.7 mM to about 3 mM, about 0.8 mM to about 3 mM, about 0.1 mM to about 2 mM, about 0.2 mM to about 2 mM, about 0.5 mM to about 2 mM, about 0.75 mM to about 2 mM, about 0.9 mM to about 2 mM, or about 1 mM to about 2 mM.

For example, the composition may have a pH of about 5.0 to about 7.5. For example, the composition may have a pH of about 5.9 to about 6.9. For example, the composition may have a pH of about 5.9 to about 6.8, about 5.9 to about 6.7, about 5.9 to about 6.6, about 5.9 to about 6.5, about 5.9 to about 6.4, about 6.0 to about 6.9, about 6.1 to about 6.9, about 6.2 to about 6.9, about 6.3 to about 6.9, about 6.4 to about 6.9, about 6.0 to about 6.5, about 6.1 to about 6.5, about 6.2 to about 6.5, about 6.3 to about 6.5, or about 6.4 to about 6.5. For example, the composition may have a pH of about 6.3 to about 6.5. For example, the composition may have a pH of about 6.4.

For example, the composition may comprise a buffering component, wherein the buffering component is selected from one or more of the group consisting of: sodium acetate-acetic acid, histidine-acetic acid, L-histidine-L-histidine hydrochloride, sodium phosphate and citric acid-sodium hydroxide.

For example, the buffering component may be at a concentration of about 5 mM to about 50 mM. For example, the buffering component may be at a concentration of about 5 mM to about 50 mM, about 5 mM to about 45 mM, about 5 mM to about 40 mM, about 5 mM to about 35 mM, about 5 mM to about 30 mM, about 5 mM to about 25 mM, about 5 mM to about 20 mM, about 5 mM to about 50 mM, or about 5 mM to about 30 mM. For example, the buffering component may be at a concentration of about 10 mM to 50 mM. For example, the buffering component may be at a concentration of about 10 mM to about 50 mM, about 10 mM to about 45 mM, about 10 mM to about 40 mM, about 10 mM to about 35 mM, about 10 mM to about 30 mM, about 10 mM to about 25 mM, about 10 mM to about 20 mM, about 20 mM to about 50 mM, or about 20 mM to about 30 mM.

For example, the buffering component may comprise sodium acetate-acetic acid, which may be at a concentration of about 10 mM to about 50 mM. For example, the sodium acetate-acetic acid may be at a concentration of about 5 mM to 50 mM, about 5 mM to about 45 mM, about 5 mM to about 40 mM, about 5 mM to about 35 mM, about 5 mM to about 30 mM, about 5 mM to about 25 mM, about 5 mM to about 20 mM, about 10 mM to 50 mM, about 10 mM to about 45 mM, about 10 mM to about 40 mM, about 10 mM to about 35 mM, about 10 mM to about 30 mM, about 10 mM to about 25 mM, about 10 mM to about 20 mM, about 20 mM to about 50 mM, or about 20 mM to about 30 mM. For example, the buffering component may comprise sodium acetate-acetic acid, which may be at a concentration of about 5 mM to about 35 mM. For example, the buffering component may comprise sodium acetate-acetic acid, which may be at a concentration of about 10 mM to about 30 mM. For example, the buffering component may comprise sodium acetate-acetic acid, which may be at a concentration of about 20 mM.

For example, the buffering component may comprise L-histidine-L-histidine hydrochloride, which may be at a concentration of about 10 mM to about 50 mM. For example, the L-histidine-L-histidine hydrochloride may be at a concentration of about 5 mM to 50 mM, about 5 mM to about 45 mM, about 5 mM to about 40 mM, about 5 mM to about 35 mM, about 5 mM to about 30 mM, about 5 mM to about 25 mM, about 5 mM to about 20 mM, about 10 mM to 50 mM, about 10 mM to about 45 mM, about 10 mM to about 40 mM, about 10 mM to about 35 mM, about 10 mM to about 30 mM, about 10 mM to about 25 mM, about 10 mM to about 20 mM, about 20 mM to about 50 mM, or about 20 mM to about 30 mM. For example, the buffering component may comprise L-histidine-L-histidine hydrochloride, which may be at a concentration of about 5 mM to about 35 mM. For example, the buffering component may comprise L-histidine-L-histidine hydrochloride, which may be at a concentration of about 10 mM to about 30 mM. For example, the buffering component may comprise L-histidine-L-histidine hydrochloride, which may be at a concentration of about 20 mM.

For example, the composition may comprise a surfactant, wherein the surfactant may be selected from one or more of the group consisting of: polysorbate 20, polysorbate 80 and poloxamer 188. The concentration of the surfactant is expressed in mg/mL or % (w/v) in the present application, and it will be understood by those skilled in the art that conversion of the two is possible. For example, 0.02% surfactant can be converted to 0.2 mg/mL.

For example, the surfactant may be at a concentration of about 0 mg/mL to about 0.8 mg/mL. For example, the surfactant may be at a concentration of about 0.05 mg/mL to about 0.8 mg/mL. For example, the surfactant may be at a concentration of about 0 mg/mL to about 0.8 mg/mL, about 0.1 mg/mL to about 0.8 mg/mL, about 0.2 mg/mL to about 0.8 mg/mL, about 0.3 mg/mL to about 0.8 mg/mL, about 0.1 mg/mL to about 0.3 mg/mL, about 0.1 mg/mL to about 0.2 mg/mL, about 0.05 mg/mL to about 0.3 mg/mL, about 0 mg/mL to about 0.4 mg/mL, about 0.05 mg/mL to about 0.4 mg/mL, about 0.05 mg/mL to about 0.2 mg/mL, about 0.2 mg/mL to about 0.4 mg/mL, or about 0 mg/mL to about 0.2 mg/mL.

For example, the surfactant may comprise polysorbate 20, which may be at a concentration of about 0 mg/mL to about 0.8 mg/mL. For example, the polysorbate 20 may be at a concentration of about 0.05 mg/mL to about 0.8 mg/mL. For example, the polysorbate 20 may be at a concentration of about 0 mg/mL to about 0.8 mg/mL, about 0.1 mg/mL to about 0.8 mg/mL, about 0.2 mg/mL to about 0.8 mg/mL, about 0.3 mg/mL to about 0.8 mg/mL, about 0.1 mg/mL to about 0.3 mg/mL, about 0.1 mg/mL to about 0.2 mg/mL, about 0.05 mg/mL to about 0.3 mg/mL, about 0 mg/mL to about 0.4 mg/mL, about 0.05 mg/mL to about 0.4 mg/mL, about 0.05 mg/mL to about 0.2 mg/mL, about 0.2 mg/mL to about 0.4 mg/mL, or about 0 mg/mL to about 0.2 mg/mL. For example, the surfactant may comprise polysorbate 20, which may be at a concentration of about 0.2 mg/mL to about 0.4 mg/mL. For example, the surfactant may comprise polysorbate 20, which may be at a concentration of about 0.2 mg/mL. For example, the surfactant may comprise polysorbate 20, which may be at a concentration of about 0.4 mg/mL.

For example, the surfactant may comprise polysorbate 80, which may be at a concentration of about 0 mg/mL to about 0.8 mg/mL. For example, the polysorbate 80 may be at a concentration of about 0.05 mg/mL to about 0.8 mg/mL. For example, the polysorbate 80 may be at a concentration of about 0 mg/mL to about 0.8 mg/mL, about 0.1 mg/mL to about 0.8 mg/mL, about 0.2 mg/mL to about 0.8 mg/mL, about 0.3 mg/mL to about 0.8 mg/mL, about 0.1 mg/mL to about 0.3 mg/mL, about 0.1 mg/mL to about 0.2 mg/mL, about 0.05 mg/mL to about 0.3 mg/mL, about 0 mg/mL to about 0.4 mg/mL, about 0.05 mg/mL to about 0.4 mg/mL, about 0.05 mg/mL to about 0.2 mg/mL, about 0.2 mg/mL to about 0.4 mg/mL, or about 0 mg/mL to about 0.2 mg/mL. For example, the surfactant may comprise polysorbate 80, which may be at a concentration of about 0.2 mg/mL to about 0.4 mg/mL. For example, the surfactant may comprise polysorbate 80, which may be at a concentration of about 0.2 mg/mL. For example, the surfactant may comprise polysorbate 80, which may be at a concentration of about 0.4 mg/mL.

For example, the surfactant may comprise poloxamer 188, and the poloxamer 188 may be at a concentration of about 0.05 mg/mL to about 5 mg/mL, about 0.08 mg/mL to about 5 mg/mL, about 0.1 mg/mL to about 5 mg/mL, about 0.2 mg/mL to about 5 mg/mL, about 0.05 mg/mL to about 3 mg/mL, about 0.1 mg/mL to about 3 mg/mL, about 0.15 mg/mL to about 3 mg/mL, about 0.2 mg/mL to about 3 mg/mL, about 0.05 mg/mL to about 2 mg/mL, about 0.075 mg/mL to about 2 mg/mL, about 0.1 mg/mL to about 2 mg/mL, about 0.15 mg/mL to about 2 mg/mL, about 0.2 mg/mL to about 2 mg/mL, about 0.3 mg/mL to about 2 mg/mL, or about 0.5 mg/mL to about 2 mg/mL.

In the present application, the composition may comprise the antigen-binding fragment described herein and the excipient described herein, and may have a pH of about 5.9 to about 6.9. For example, the composition may comprise the antigen-binding fragment described herein, the excipient described herein and the buffering component described herein, and may have a pH of about 5.9 to about 6.9. For example, the composition may comprise the antigen-binding fragment described herein, the excipient described herein, the buffering component described herein and the surfactant described herein, and may have a pH of about 5.9 to about 6.9.

In the present application, the composition may comprise the antigen-binding fragment described herein and proline, and may have a pH of about 5.9 to about 6.9. For example, the composition may comprise the antigen-binding fragment described herein, proline and sodium acetate-acetic acid, and may have a pH of about 5.9 to about 6.9. For example, the composition may comprise the antigen-binding fragment described herein, proline, sodium acetate-acetic acid and polysorbate 20, and may have a pH of about 5.9 to about 6.9. For example, the composition may comprise the antigen-binding fragment described herein, proline, sodium acetate-acetic acid and polysorbate 20, and may have a pH of about 6.3 to about 6.5.

For example, the composition may comprise: 1) about 20 mg/mL to about 220 mg/mL of the antigen-binding fragment described herein; 2) about 10 mM to about 50 mM of sodium acetate-acetic acid; 3) about 50 mM to about 500 mM of proline; and 4) about 0.05 mg/mL to about 0.8 mg/mL of polysorbate 20; and the composition may have a pH of about 5.9 to about 6.9.

For example, the composition may consist of: 1) about 20 mg/mL to about 220 mg/mL of the antigen-binding fragment described herein; 2) about 10 mM to about 50 mM of sodium acetate-acetic acid; 3) about 50 mM to about 500 mM of proline; and 4) about 0.05 mg/mL to about 0.8 mg/mL of polysorbate 20; and the composition may have a pH of about 5.9 to about 6.9.

For example, the composition may comprise: 1) about 20 mg/mL to about 220 mg/mL of the antigen-binding fragment described herein; 2) about 5 mM to about 35 mM of sodium acetate-acetic acid; 3) about 50 mM to about 390 mM of proline; and 4) about 0 mg/mL to about 0.4 mg/mL of polysorbate 20; and the composition may have a pH of about 5.9 to about 6.9.

For example, the composition may consist of: 1) about 20 mg/mL to about 220 mg/mL of the antigen-binding fragment described herein; 2) about 5 mM to about 35 mM of sodium acetate-acetic acid; 3) about 50 mM to about 390 mM of proline; and 4) about 0 mg/mL to about 0.4 mg/mL of polysorbate 20; and the composition may have a pH of about 5.9 to about 6.9.

For example, the composition may comprise: 1) about 50 mg/mL to about 200 mg/mL of the antigen-binding fragment described herein; 2) about 10 mM to about 30 mM of sodium acetate-acetic acid; 3) about 150 mM to about 250 mM of proline; and 4) about 0.2 mg/mL to about 0.4 mg/mL of polysorbate 20; and the composition may have a pH of about 6.3 to about 6.5.

For example, the composition may consist of: 1) about 50 mg/mL to about 200 mg/mL of the antigen-binding fragment described herein; 2) about 10 mM to about 30 mM of sodium acetate-acetic acid; 3) about 150 mM to about 250 mM of proline; and 4) about 0.2 mg/mL to about 0.4 mg/mL of polysorbate 20; and the composition may have a pH of about 6.3 to about 6.5.

For example, the composition may comprise: 1) about 200 mg/mL of the antigen-binding fragment described herein; 2) about 20 mM of sodium acetate-acetic acid; 3) about 220 mM of proline; and 4) about 0.2 mg/mL of polysorbate 20; and the composition may have a pH of about 6.4.

For example, the composition may consist of: 1) about 200 mg/mL of the antigen-binding fragment described herein; 2) about 20 mM of sodium acetate-acetic acid; 3) about 220 mM of proline; and 4) about 0.2 mg/mL of polysorbate 20; and the composition may have a pH of about 6.4.

In the present application, the composition may be used for injection. In the present application, the composition may be used for subcutaneous injection. For example, the composition may also be used for subcutaneous injection, intramuscular injection, intravenous injection, parenteral administration or intravenous infusion.

In the present application, the composition may be a formulation. For example, the composition may be a liquid formulation.

In another aspect, the present application provides a kit comprising the composition described herein and a container holding the composition described herein.

For example, the container may comprise a vial (e.g., a glass vial), an ampoule, a syringe, an injection pen and/or an IV bag. In the present application, the container may be sterile. For example, the container may comprise a glass vial.

For example, the kit may comprise a delivery device that can administer the composition described herein. For example, the delivery device may comprise a vial, an ampoule, a syringe, an injection pen and/or an IV bag.

In the present application, the composition in the container may have a volume of about 0.5 mL to about 5.0 mL. For example, the composition in the container may have a volume of about 0.5 mL to about 1.5 mL. For example, the composition may have a volume of about 1.5 mL. For example, the composition may have a volume of about 1.0 mL. In the present application, in the container, the antigen-binding fragment may be at a concentration of about 200 mg/1.0 mL/vial or about 300 mg/1.5 mL/vial.

In another aspect, the present application provides use of the composition described herein and/or the kit described herein in the preparation of a medicament for preventing, alleviating and/or treating a tumor.

The present application provides use of the composition described herein and/or the kit described herein in preventing, alleviating and/or treating a tumor.

The present application provides a method for preventing, alleviating and/or treating a tumor, which comprises the following step: administering the composition described herein and/or the kit described herein to a subject in need thereof.

In the present application, the tumor may comprise a PD-L1 positive tumor. For example, the tumor may comprise a malignant solid tumor.

In the present application, the tumor may comprise a tumor with microsatellite instability (MSI-H)/deficient mismatch repair (dMMR) (e.g., a MSI-H/dMMR pan-tumor solid tumor). For example, the tumor may comprise an advanced colorectal cancer or gastric cancer with microsatellite instability (MSI-H)/deficient mismatch repair (dMMR). For example, the tumor may comprise a biliary tract cancer that is not suitable for surgical resection or is metastatic, and/or an untreated gastric or gastroesophageal junction adenocarcinoma that is not suitable for resection or is metastatic.

In another aspect, the present application also provides use of the composition described herein and/or the kit described herein in the preparation of a medicament for preventing, alleviating and/or treating an infectious disease.

The present application provides use of the composition described herein and/or the kit described herein in preventing, alleviating and/or treating an infectious disease.

The present application provides a method for preventing, alleviating and/or treating an infectious disease, which comprises the following step: administering the composition described herein and/or the kit described herein to a subject in need thereof.

In the present application, the infectious disease comprises a disease caused by a viral infection, a bacterial infection, a fungal infection and/or a parasitic infection.

The present application also provides a method for producing the composition described herein and/or the kit described herein. The method may comprise mixing, in any order, any relevant components necessary to prepare the composition described herein. For example, the method may comprise preparing a premix (or a premixed solution) of the excipient, the buffering component and/or the surfactant. For example, the method may comprise forming a buffering system. The buffering system may comprise the buffering component described herein. The method described herein may comprise removing particulate matters (e.g., by filtration). Without being bound by any theory, the following examples are intended only to illustrate the formulations, preparation methods, use, etc., of the present application, but not to limit the scope of the present application.

### Examples

Detection items and methods:
(1) Endogenous fluorescence spectroscopy: the fluorescence spectra of amino acids (tryptophan, tyrosine and phenylalanine) in the protein molecule capable of exhibiting fluorescence endogenously in polar and non-polar environments are used. If the fluorescence spectrum is red-shifted, that is, the maximum emission wavelength changes, the consistency of the higher-order structures of the protein, i.e., the mutual transformation of the higher-order structures, can be determined. The samples were diluted to 40 mg/mL, and a high throughput intrinsic fluorescence spectrometer optim1000 (Pall) was used. The temperature range of the test was 20-95 °C and the test interval was 1 °C. The aggregation tendency of samples were determined based on results of SLS (static light scattering) at a wavelength of 473 nm, and the denaturation temperature was a value measured at BCM (barycentric mean). The evaluating was performed based on Tm values (temperature of half denaturation) calculated according to the formula.
(2) SEC-HPLC (size-exclusion chromatography): reference was made to Size-Exclusion Chromatography (General Rules 0514, Volume III of Chinese Pharmacopoeia 2015*)* for detecting monomer content of the product as well as polymers and fragments. The chromatographic column was TOSOH G3000 7.8 × 300 mm, 5 µm; the mobile phase was 20 mM Na₂HPO₄, 300 mM NaCl; the detection wavelength was 280 nm.
(3) WCX-HPLC (weak cation exchange chromatography): reference was made to High Performance Liquid Chromatography (General Rules 0512, Volume III of Chinese Pharmacopoeia 2015*)* for detecting the content of principal component, the acidic component and basic component of the product. The chromatographic column was Propac WCX-10 4 × 250 mm, 10 µm; the mobile phase was 10 mM Na₂HPO₄, 10 mM Na₂HPO₄ + 500 mM NaCl; the detection wavelength was 280 nm.
(4) Binding and blocking activity: reference was made to the test methods described in Examples 5.2 and 5.3 of WO 2017/020802A1.
(5) Viscosity: the viscosity was measured in an automatic mode using microVISC, a portable microviscometer of rheosense in U.S.
(6) Osmotic pressure: the osmotic pressure was detected using an osmolarity method. Instruments: an osmometer; reagents: 300 mosm/Kg calibration solution, 900 mosm/Kg calibration solution and sodium chloride
   Consumable materials: centrifuge tubes, dust-free paper, etc.;
   Experimental procedures: 100 µL of the sample was added into a sample tube, and it should be ensured that no bubbles were generated in the sample tube; the sample tube was placed on the detection probe after the sample probe and the secondary probe were wiped by using dust-free paper; the detection probe was lowered down and the sample tube was in a cold trap, and the "Enter" key was pressed to start the test. The test sample was subjected to parallel assay (assayed 2 times). Zero point calibration: within ±2; 300-point calibration, within ±4; and 900-point calibration: within ±8.
(7) CE-SDS purity: after being denatured (non-reduced condition) or denatured and reduced (reduced condition), the sample was put into a capillary electrophoresis apparatus for electrophoretic analysis, and the calculation was carried out according to an area normalization method.
(8) Protein content: reference was made to Method 6 of Determination of Protein Content (General Rules 0731, Volume III of Chinese Pharmacopoeia 2015*),* and the protein content was calculated according to the absorbance value of protein at 280 nm wavelength and the theoretical extinction coefficient of the protein by using the micro-ultraviolet spectrophotometer Nanodrop 2000.
(9) Measurement of turbidity: the absorbance value of the sample at 340 nm was measured on the Agilent 8453 spectrophotometer.
(10) Insoluble particles: the number of particles ≥ 10 µm and ≥ 25 µm in the sample was measured on the AccuSizer 780SIS insoluble microparticle analyzer.
(11) Dynamic Light Scattering (DLS): the average particle diameter (Z-average diameter) and the distribution coefficient (PDI) were measured on a PUNK-type particle-size analyzer.

Preparation of antigen-binding protein:
The antigen-binding protein comprises an immunoglobulin single variable domain capable of specifically binding to PD-L1, and the immunoglobulin single variable domain may comprise an amino acid sequence set forth in SEQ ID NO: 2. Hereinafter, the antigen-binding protein is referred to as "PD-L1 monoclonal antibody". The PD-L1 monoclonal antibody was obtained by a method of artificially synthesizing amino acid sequences. The PD-L1 monoclonal antibody comprises an amino acid sequence set forth in any one of SEQ ID NOs: 14-31, in particular an amino acid sequence set forth in SEQ ID NO: 21.

The proline referred to in the Examples includes L-proline.

### Example 1

The following formulation formulas are obtained according to Table 1:

**Table 1. Different compositions of formulation formulas in screening experiment**

| Formula | Content of PD-L1 monoclonal antibody (mg/mL) | Buffering system | Excipient | Surfactant | pH |
|---|---|---|---|---|---|
| 1 | 200±20 | 20 mM of sodium acetate-acetic acid | 250 mM of glycine | 0.02% polysorbate 20 | 6.5 |
| 2 | 200±20 | 20 mM of sodium acetate-acetic acid | 250 mM of glycine | 0.02% polysorbate 20 | 7.0 |
| 3 | 200±20 | 20 mM of sodium acetate-acetic acid | 250 mM of L-proline | 0.02% polysorbate 20 | 6.5 |
| 4 | 200±20 | 20 mM of sodium acetate-acetic acid | 250 mM of L-proline | 0.02% polysorbate 20 | 7.0 |
| 5 | 200±20 | 20 mM of sodium phosphate | 250 mM of glycine | 0.02% polysorbate 20 | 6.5 |
| 6 | 200±20 | 20 mM of sodium phosphate | 250 mM of glycine | 0.02% polysorbate 20 | 7.0 |
| 7 | 200±20 | 20 mM of sodium phosphate | 250 mM of L-proline | 0.02% polysorbate 20 | 6.5 |
| 8 | 200±20 | 20 mM of sodium phosphate | 250 mM of L-proline | 0.02% polysorbate 20 | 7.0 |
| 9 | 200±20 | 20 mM of sodium phosphate | 250 mM of glycine | 0.02% polysorbate 20 | 6.8 |
| 10 | 200±20 | 20 mM of L-histidine-acetic acid | 250 mM of glycine | 0.02% polysorbate 20 | 7.0 |
| 11 | 200±20 | -- | 250 mM of glycine | 0.02% polysorbate 20 | 7.0 |
| 12 | 200±20 | -- | 250 mM of L-proline | 0.02% polysorbate 20 | 7.0 |
| 13 | 200±20 | 20 mM of sodium phosphate | 150 mM of L-arginine hydrochloride | 0.02% polysorbate 20 | 7.0 |
| 14 | 200±20 | 20 mM of sodium phosphate | 90 mg/mL of sucrose | 0.02% polysorbate 20 | 7.0 |
| 15 | 200±20 | 20 mM of sodium phosphate | 150 mM of L-arginine hydrochloride | 0.2% poloxamer 188 | 7.0 |
| 16 | 50±2 | 20 mM of sodium phosphate | 90 mg/mL of sucrose | 0.02% polysorbate 20 | 7.0 |
| 17 | 50±2 | 20 mM of sodium phosphate | 150 mM of L-arginine hydrochloride | 0.02% polysorbate 20 | 7.0 |
| 18 | 50±2 | 20 mM of sodium phosphate | 250 mM of glycine | 0.02% polysorbate 20 | 7.0 |
| 19 | 50±2 | 20 mM of sodium phosphate | 50 mg/mL of mannitol | 0.02% polysorbate 20 | 7.0 |

| | | | | | |
|---|---|---|---|---|---|
| Note: - -represents not added | | | | | |

The formulation formulas in the Table 1 are screened and detected according to Table 2.

**Table 2. Detection items for screening of formulation formulas**

| Detection items | Duration of investigation (day) (temperature: 40±2 °C) | | |
|---|---|---|---|
| | 0 | 14 | 28 |
| Endogenous fluorescence spectra | √ | -- | -- |
| SEC-HPLC | √ | √ | √ |
| WCX-HPLC | √ | √ | √ |
| Viscosity | √ | -- | -- |
| Activity | √ | -- | √ |
| Osmotic pressure | √ | -- | -- |

| | | | |
|---|---|---|---|
| 1) Detection of endogenous fluorescence spectra | | | |

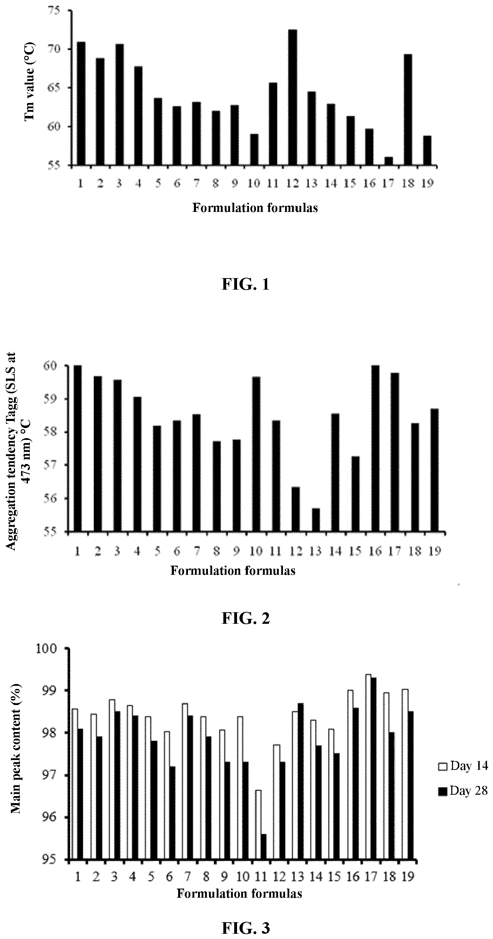

The Tm values and the detection results of the aggregation tendency corresponding to formulas 1-19 are shown in FIG. 1 and FIG. 2, respectively.

The results of FIG. 1 show that formulas 1-4 and 12 were more structurally stable; the results in FIG. 2 show that formulas 1-4 and 10 were less prone to form aggregates. It can be seen that in this detection, the stability of formulas 1-4 was very good, that is, the use of sodium acetate-acetic acid as the buffering system correlates with further improvement in stability.

Comparing the results of formulas 1-2 and formulas 3-4 separately, it can be seen that lower pH correlates with further improvement in stability.

### 2) SEC-HPLC detection of main peak content

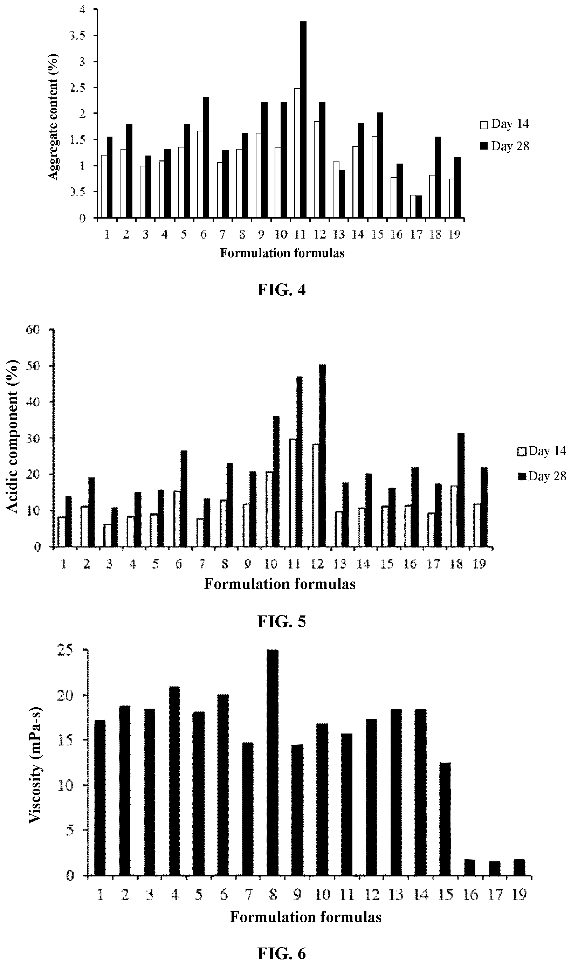

The results of the main peak content and the results of the aggregate content corresponding to formulas 1-19 as detected by SEC-HPLC are shown in FIG. 3 and FIG. 4, respectively.

The results show that under the condition of 28 days of high temperature, the purity of the PD-L1 monoclonal antibody in all the formulas except for formula 11 was higher than 97%, which shows that a buffering system is necessary.

Comparing the results of formulas 1, 3, 5 and 7 and formulas 2, 4, 6 and 8, respectively, it can be seen that lower pH correlates with further improvement in formula stability.

Comparing the stability of formulas 1 and 3 (pH 6.5, sodium acetate-acetic acid as the buffering system) and formulas 5 and 7 (pH 6.5, sodium phosphate as the buffering system) separately, it is found that the presence of L-proline correlates with further improvement in stability. Comparing the results of formulas 6 and 8 and formulas 13 and 14 separately, it is found that the inclusion of L-arginine or the inclusion of L-proline correlates with further improvement in stability.

Comparing the formulas 2, 6 and 10, it is found that the use of sodium acetate-acetic acid as the buffering system correlates with further improvement in stability.

Comparing the results of formulas 14 and 16, formulas 13 and 17 and formulas 6 and 18 separately, it is found that the stability of the formulas seemed to be unaffected whether the content of the PD-L1 monoclonal antibody was 50 mg/mL or 200 mg/mL.

### 3) WCX-HPLC detection of acidic component content

The results for acidic component content detected by WCX-HPLC are shown in FIG. 5, which can demonstrate the trend of change in the acidic component (which may include components generated by decomposition of the PD-L1 monoclonal antibody under high temperature conditions) in the formulas.

Comparing the results of formulas 1, 3, 5 and 7 and formulas 2, 4, 6 and 8, respectively, it can be seen that lower pH correlates with further reduction of the acidic component of the formulas. Comparing the acidic components of formula 1 and formula 5, formula 3 and formula 7 and formulas 2, 6 and 10 separately, it is found that the use of sodium acetate-acetic acid as the buffering system correlates with further reduction in the acidic components of the formulas. Comparing the acidic components of formulas 6, 8, 13 and 14, it is found that the inclusion of L-proline correlates with further reduction in the acidic components of the formulas. Comparing the results of formulas 14 and 16, formulas 13 and 17 and formulas 6 and 18 separately, it can be seen that the acidic component content of the formulas seemed to be unaffected whether the content of the PD-L1 monoclonal antibody was 50 mg/mL or 200 mg/mL.

### 4) Viscosity detection

The results of the viscosity detection are shown in FIG. 6. Except for the formula 8, the viscosity of other formulas all met the requirement of subcutaneous injection on viscosity.

Comparing the results of formulas 14 and 16 and formulas 13 and 17, separately, it can be seen that when the content of the PD-L1 monoclonal antibody was 200 mg/mL, the viscosity was increased compared to the situation when the content of the PD-L1 monoclonal antibody was 50 mg/mL, but administration by subcutaneous injection was not affected.

### 5) Detection of activity

Formula 1 and formula 3 were selected for activity assay under high temperature treatment.

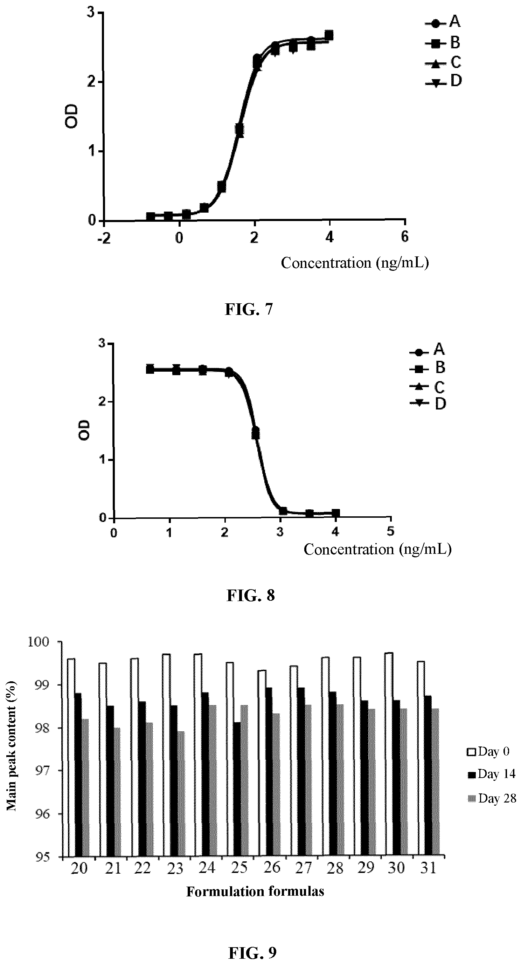

The results of the binding activity and blocking activity of both are shown in FIG. 7, FIG. 8 and Table 3. The A-D in FIGs. 7-8 represent the results on day 0 of formula 1, day 0 of formula 3, day 28 of formula 1 and day 28 of formula 3, respectively. The results show that after 28 days of high-temperature treatment, the activity of formula 1 and formula 3 was not significantly different, and the activity of each of the two was not significantly different from its activity on day 0.

**Table 3. Binding activity and blocking activity of formula 1 and formula 3**

| | Formula 1 | | Formula 3 | |
|---|---|---|---|---|
| | Day 0 | Day 28 | Day 0 | Day 28 |
| Binding activity EC50 | 39.25 | 42.57 | 39.98 | 39.35 |
| Blocking activity EC50 | 401.8 | 389.8 | 388.9 | 388.3 |

### Example 2

Based on the formula screening in Example 1, the influence of factors on the formula performance is further examined, and the formulation formulas for study are shown in Table 4.

**Table 4. Different compositions of formulation in formula optimization**

| Formula | Kinds for examination | Content of PD-L1 monoclonal antibody | Buffer | Stabilizer | Surfactant | pH |
|---|---|---|---|---|---|---|
| 19 A | Surfactant type | 200mg/mL | 20 mM of sodium acetate-acetic acid | 220 mM of L-proline | 0.02% polysorbate 20 | 6.4 |
| 20 A | | | | | 0.02% polysorbate 80 | |
| 21 A | | | | | 0.02% poloxamer P188 | |
| 22 A | Buffer type | | 20 mM of L-histidine-acetic acid | | 0.02% polysorbate 20 | |
| 23 A | | | 20 mM of L-histidine-L-histidine hydrochloric acid | | | |
| 24 A | | | 20 mM of sodium phosphate | | | |
| 25 A | | | 20 mM of citric acid-sodium hydroxide | | | |
| 26 A | Stabilizer type | | 20 mM of sodium acetate-acetic acid | 90 mg/mL of sucrose | | |
| 27 A | | | | 250 mM of glycine | | |
| 28 A | | | | 150 mM of L-arginine hydrochloride | | |
| 29 A | | | | 150 mM of sodium chloride | | |
| 30 A | PD-L1 monoclonal antibody | | NA | NA | NA | NA |
| 31 A | PD-L1 monoclonal antibody + stabilizer | | NA | 220 mM of L-proline | NA | NA |
| 32 A | PD-L1 monoclonal antibody + buffer + stabilizer | | 20 mM of sodium acetate-acetic acid | 220 mM of L-proline | NA | 6.4 |
| 33 A | Content of PD-L1 monoclonal antibody | 20mg/mL | 20 mM of sodium acetate-acetic acid | 220 mM of L-proline | 0.02% polysorbate 20 | 6.4 |
| 34 A | | 220mg/mL | 20 mM of sodium acetate-acetic acid | 220 mM of L-proline | 0.02% polysorbate 20 | 6.4 |

| | | | | | | |
|---|---|---|---|---|---|---|
| Note: NA -represents not added | | | | | | |

After the formulations were subjected to acceleration for 14 days and 28 days at high temperature (40±2 °C), the purity of the PD-L1 monoclonal antibody detected by SEC-HPLC and the change in the acidic and basic components detected by WCX-HPLC were evaluated, and the viscosity of samples on day 0 was detected. Other supplementary experimental investigation and evaluation items for formulation composition are shown in Table 5.

**Table 5. Detection items for optimization of formulation formulas**

| Detection items | Duration of investigation (day) (temperature: 40±2 °C) | | |
|---|---|---|---|
| | 0 | 14 | 28 |
| SEC-HPLC | √ | √ | √ |
| WCX-HPLC | √ | √ | √ |
| Viscosity | √ | -- | -- |

The SEC-HPLC detection results are shown in Table 6.

**Table 6. SEC-HPLC detection results of study on composition of formulation formulas**

| Formula | Kinds for examination | Day 0 (%) | | | Day 14 (%) | | | Day 28 (%) | | |
|---|---|---|---|---|---|---|---|---|---|---|
| | | Aggregate | Monomer | Fragment | Aggregate | Monomer | Fragment | Aggregate | Monomer | Fragment |
| 19 A | Surfactant type | 0.5 | 99.5 | 0 | 0.8 | 99.0 | 0.2 | 1.1 | 98.6 | 0.3 |
| 20 A | | 0.5 | 99.5 | 0 | 0.8 | 99.0 | 0.2 | 1.2 | 98.5 | 0.3 |
| 21 A | | 0.4 | 99.6 | 0 | 0.8 | 99.0 | 0.2 | 1.1 | 98.6 | 0.3 |
| 22 A | Buffer type | 0.3 | 99.7 | 0 | 0.6 | 99.2 | 0.2 | 0.8 | 98.9 | 0.3 |
| 23 A | | 0.3 | 99.7 | 0 | 0.6 | 99.2 | 0.2 | 0.8 | 98.9 | 0.3 |
| 24 A | | 0.4 | 99.6 | 0 | 0.9 | 98.9 | 0.2 | 1.2 | 98.4 | 0.4 |
| 25 A | | 0.4 | 99.5 | 0.1 | 0.7 | 99.1 | 0.2 | 0.9 | 98.8 | 0.3 |
| 26 A | Stabilizer | 0.5 | 99.5 | 0 | 0.9 | 98.9 | 0.2 | 1.2 | 98.5 | 0.3 |
| 27 A | type | 0.4 | 99.6 | 0 | 1.0 | 98.8 | 0.2 | 1.4 | 98.3 | 0.3 |
| 28 A | | 0.4 | 99.6 | 0 | 0.8 | 99.0 | 0.2 | 1.0 | 98.7 | 0.3 |
| 29 A | | 0.5 | 99.5 | 0 | 1.0 | 98.8 | 0.2 | 1.4 | 98.3 | 0.3 |
| 33 A | Content of PD-L1 monoclonal antibody | 0.3 | 99.7 | 0 | 0.3 | 99.6 | 0.1 | 0.3 | 99.4 | 0.3 |
| 34 A | | 0.5 | 99.5 | 0 | 0.9 | 99.0 | 0.2 | 1.1 | 98.6 | 0.3 |

The results of the WCX-HPLC detection are shown in Table 7.

**Table 7. WCX-HPLC detection results of study on composition of formulation formulas**

| Formula | Kinds for examination | Day 0 (%) | | | Day 14 (%) | | | Day 28 (%) | | |
|---|---|---|---|---|---|---|---|---|---|---|
| | | Acidic component | Principal component | Basic component | Acidic component | Principal component | Basic component | Acidic component | Principal component | Basic component |
| 19A | Surfactant type | 4.4 | 92.2 | 3.4 | 7.7 | 87.7 | 4.6 | 10.7 | 83.4 | 5.9 |
| 20 A | | 4.5 | 91.9 | 3.6 | 7.7 | 87.3 | 5.0 | 10.7 | 83.2 | 6.1 |
| 21A | | 4.4 | 92.0 | 3.6 | 7.8 | 87.0 | 5.1 | 10.7 | 83.9 | 5.5 |
| 22 A | Buffer type | 4.5 | 91.9 | 3.6 | 8.0 | 86.9 | 5.2 | 11.1 | 83.0 | 5.9 |
| 23 A | | 4.4 | 91.9 | 3.7 | 7.7 | 87.3 | 5.0 | 10.8 | 83.3 | 6.0 |
| 24 A | | 4.3 | 92.7 | 3.0 | 9.2 | 84.3 | 6.5 | 13.1 | 79.6 | 7.2 |
| 25 A | | 4.3 | 92.1 | 3.7 | 7.0 | 85.9 | 7.1 | 9.2 | 82.0 | 8.8 |
| 26 A | Stabilizer type | 4.4 | 92.0 | 3.7 | 6.6 | 88.4 | 5.0 | 8.5 | 85.3 | 6.2 |
| 27 A | | 4.4 | 90.9 | 4.8 | 9.5 | 85.5 | 4.9 | 13.3 | 81.0 | 5.7 |
| 28 A | | 5.7 | 90.9 | 3.4 | 7.4 | 87.0 | 5.7 | 10.0 | 83.4 | 6.6 |
| 29 A | | 4.3 | 92.2 | 3.5 | 6.3 | 88.5 | 5.2 | 7.6 | 85.9 | 6.5 |
| 33 A | Content of PD-L1 monoclonal antibody | 4.4 | 92.0 | 3.6 | 8.4 | 86.5 | 5.1 | 11.4 | 82.7 | 5.9 |
| 34 A | | 4.3 | 92.0 | 3.7 | 7.6 | 87.2 | 5.1 | 10.1 | 84.1 | 5.7 |

### (1) Study on influence of surfactant type on stability of formulation

The content of the PD-L1 monoclonal antibody was 200 mg/mL, 20 mM of sodium acetate-acetic acid was used as the buffer, 220 mM of proline (L-proline) was used as the stabilizer, and 0.02% polysorbate 20, polysorbate 80 and poloxamer P188 were selected as surfactants. The target protein was detected by SEC-HPLC and WCX-HPLC when the formulations were subjected to acceleration of 0 days, 14 days and 28 days, so as to examine the influence of different surfactants on the stability of the formulations (see formulation formulas 19A, 20A and 21A). The study results show that polysorbate 20, polysorbate 80 and poloxamer P188 can be used as the surfactant of the PD-L1 monoclonal antibody formulation.

### (2) Study on influence of stabilizer type on stability of formulation

The content of the PD-L1 monoclonal antibody was 200 mg/mL, 20 mM of sodium acetate-acetic acid was used as the buffer, 0.02% polysorbate 20 was used as the surfactant, the pH was 6.4, and 220 mM of L-proline, 90 mg/mL of sucrose, 250 mM of glycine, 150 mM of L-arginine hydrochloride and 150 mM of sodium chloride were selected as stabilizers. The target protein was detected by SEC-HPLC and WCX-HPLC when the formulations were subjected to acceleration of 0 days, 14 days and 28 days, so as to examine the influence of different stabilizers on the stability of the formulations (see formulation formulas 19A, 26A, 27A, 28A and 29A). The study results show that L-proline, sucrose, glycine, L-arginine hydrochloride or sodium chloride can be used as the stabilizer of the PD-L1 monoclonal antibody formulation.

### (3) Influence of type of buffering component on stability of formulation

The content of the PD-L1 monoclonal antibody was 200 mg/mL, 220 mM L-proline was used as the stabilizer, 0.02% polysorbate 20 was used as the surfactant, the pH was 6.4, and 20 mM of sodium acetate-acetic acid, L-histidine-acetic acid, L-histidine-L-histidine hydrochloric acid, sodium phosphate and citric acid-sodium hydroxide were selected as the buffers. The target protein was detected by SEC-HPLC and WCX-HPLC when the formulations were subjected to acceleration of 0 days, 14 days and 28 days, so as to examine the influence of different buffers on the stability of the formulations (see formulation formulas 19A, 22A, 23A, 24A and 25A). The study results show that the buffers sodium acetate-acetic acid, L-histidine-acetic acid, L-histidine-hydrochloric acid, sodium phosphate, citric acid-sodium hydroxide, etc. can be used as the buffering component of the PD-L1 monoclonal antibody formulation. In particular, the SEC-HPLC detection results of the formulas containing these buffering systems were not significantly different; WCX-HPLC results show that after 28 days of high-temperature acceleration, the principal component content of the formula containing sodium phosphate and that of the formula containing citric acid-sodium hydroxide system were slightly lower, and the principal component content of formulas containing the rest buffering systems was close.

### (4) Influence of concentration of PD-L1 monoclonal antibody on stability of formulation

Study results of the formulation formulas 33A, 34A and 19A show that formulations containing PD-L1 monoclonal antibody at a concentration of 20 mg/mL-220 mg/mL all met the requirements of formulation stability under certain conditions involving buffer, stabilizer, surfactant and/or pH.

The results of the viscosity detection on day 0 are shown in Table 8. The results show that: except that the formula 33A with low PD-L1 monoclonal antibody concentration had a relatively low viscosity, the viscosity of other formulation formulas was not significantly different and was all within an acceptable range overall. This means that formulations containing a relatively high concentration (e.g., 220 mg/mL) of PD-L1 monoclonal antibody have viscosity characteristics suitable for drug preparation (e.g., suitable for injection, such as subcutaneous injection). It can be seen that the compositions described herein have a suitable viscosity, thereby facilitating easy (e.g., subcutaneous injection) and rapid (e.g., reducing the number of injections and greatly reducing the administration time) administration, and simultaneously improving the compliance and accessibility of the administration for the patient and improving the quality of life for the patient.

**Table 8. Results of viscosity detection in study on formulation composition**

| Formula | Viscosity (mPa.s) |
|---|---|
| 19 A | 18.200 |
| 20 A | 18.270 |
| 21 A | 18.470 |
| 22 A | 17.080 |
| 23 A | 17.250 |
| 24 A | 16.390 |
| 25 A | 20.480 |
| 26 A | 22.980 |
| 27 A | 17.440 |
| 28 A | 17.280 |
| 29 A | 19.690 |
| 33 A | 1.239 |
| 34 A | 22.640 |

### Example 3

Formulation formulas were further optimized based on the results of Examples 1 and 2, and further screening was done for the pH, buffering systems, stabilizers and surfactants of formulas.

### 1. Screening for pH

Different formulas containing 200±12 mg/mL of PD-L1 monoclonal antibody within a pH range of 5.0-7.5 were tested for stability at high temperature of 40±2 °C. The purity difference of the protein was detected by SEC-HPLC and WCX-HPLC, and the particle and uniformity difference of the protein were detected by insoluble microparticles and DLS. Specific formula composition and the detection results are summarized in Table 9.

**Table 9. Detection results of 28 days of acceleration at high temperature of 40±2 °C within a pH range of 4.5-7.5**

| Content of PD-L1 monoclonal antibody | Other components | pH | SEC-HPLC monomer (%) | WCX-HPLC principal Components (%) | Insoluble microparticle | | DLS | |
|---|---|---|---|---|---|---|---|---|
| | | | | | 10µm | 25µm | Z.Av. Diameter (nm) | PDI |
| 200±12 mg/mL | 20 mM of sodium acetate-acetic acid, 220 mM of L-proline, 0.02% polysorbate 20 | 4.5 | Not detected, the solution being colloidal | | | | | |
| | | 5.0 | 97.3 | 83.7 | 43 | 8 | 16.41 | 3.28 |
| | | 5.5 | 98.3 | 85.5 | 23 | 5 | 6.74 | 0.88 |
| | | 6.4 | 98.4 | 83.3 | 18 | 3 | 10.29 | 0.37 |
| | 20 mM of PB, 220 mM of L-proline, 0.02% polysorbate 20 | 7.5 | 96.3 | 55.8 | NA | NA | 28.37 | 1.35 |

| | | | | | | | | |
|---|---|---|---|---|---|---|---|---|
| "NA" represents not detected | | | | | | | | |

SEC-HPLC detection results show that the monomer content was significant reduced under the conditions of pH 5.0 and pH 7.5; the WCX-HPLC detection results show that the principal component content was significantly reduced under the condition of pH 7.5. The detection results of insoluble particles show that the insoluble particles were less under the condition of lower pH, and the number of the insoluble particles tended to be further reduced along with the increase of the pH. The PDI index reflects the homogeneity of the solution, and the smaller the value, the better the uniformity. The results show that the solution uniformity was the best at pH 6.4, and the PDI value of the solution showed a trend of decreasing first and then increasing along with the increase of the pH.

Based on the above results, the stability of the PD-L1 monoclonal antibody within the pH range of 6.1-6.7 was further examined. Different formulas containing 200±12 mg/mL of PD-L1 monoclonal antibody were subjected to high temperature of 40±2 °C, the purity difference of the protein was detected by SEC-HPLC and WCX-HPLC, and the OD values at 340 nm were compared to detect the turbidity difference. Specific formula composition and the detection results are summarized in Table 10.

**Table 10. Detection results of 28 days of acceleration at high temperature of 40±2 °C within a pH range of 6.1-6.7**

| Content of PD-L1 monoclonal antibody | Other components | pH | SEC-HPLC monomer (%) | WCX-HPLC principal component (%) | OD₃₄₀ₘₙ |
|---|---|---|---|---|---|
| 200±12 mg/mL | 20 mM of sodium acetate-acetic acid, 220 mM of L-proline, 0.02% polysorbate 20 | 6.1 | 98.5 | 87.0 | 0.6343 |
| | | 6.3 | 98.5 | 86.3 | 0.5763 |
| | | 6.5 | 98.3 | 85.3 | 0.4717 |
| | | 6.7 | 98.5 | 84.0 | 0.4982 |

Within the pH range of 6.1-6.7, the SEC-HPLC results show no significant difference between different formulas; the WCX-HPLC results show that the principal component content tended to decrease slightly as pH increased; the turbidity results detected at OD_{340 nm} tended to decrease first and then increase along with the increase of pH.

### 2. Selection of stabilizers and buffering systems

Sodium acetate-acetic acid, L-histidine-L-histidine hydrochloride and citrate buffering systems were selected and combined with different stabilizers to further investigate the effect of the stabilizers. Stabilizers evaluated included sucrose, trehalose and maltose as sugars, L-proline, L-arginine hydrochloride and methionine as amino acids, and mannitol, sorbitol and glycerol as alcohols; sodium chloride was used as a control for simultaneous comparison.

The purity difference of the protein was detected by SEC-HPLC and WCX-HPLC, and the particle and uniformity difference between different formulas were detected by insoluble microparticles and DLS. Specific formula composition and the detection results are shown in Tables 11-13.

**Table 11. Detection results for different stabilizers after 28 days of acceleration at high temperature of 40±2 °C in the presence of the buffer sodium acetate-acetic acid**

| Content of PD-L1 monoclonal antibody | Other components | Stabilizer | SEC-HPL C monomer (%) | WCX-HPL C principal component (%) | Insoluble microparticle | | DLS | | Viscosity (cp 25 °C) |
|---|---|---|---|---|---|---|---|---|---|
| | | | | | 10µm | 25µm | Z.Av. Diameter (nm) | PDI | |
| 200±12 mg/mL | 20 mM of sodium acetate-acetic acid pH 6.4±0.1 0.02% polysorbate 20 | 220 mM of L-proline | 98.5 | 83.7 | 18 | 3 | 10.74 | 0.14 | 17.70 |
| | | 50 mg/mL of sucrose | 98.4 | 85.0 | 40 | 8 | 12.14 | 0.29 | 20.64 |
| | | 20 mg/mL of sucrose | 98.3 | 84.5 | 13 | 5 | 11.22 | 0.22 | 18.90 |
| | | 150 mM of L-arginine hydrochloride | 98.3 | 83.2 | 18 | 5 | 16.91 | 0.17 | 17.36 |
| | | 50 mM of L-arginine hydrochloride | 98.6 | 84.5 | 20 | 5 | 16.1 | 0.2 | 17.24 |
| | | 50 mg/mL of mannitol | 98.4 | 85.2 | 15 | 5 | 13.27 | 0.22 | 21.04 |
| | | 20 mg/mL of mannitol | 98.4 | 85.2 | 20 | 5 | 10.96 | 0.2 | 17.82 |
| | | 40 mg/mL of sorbitol | 98.4 | 84.9 | 30 | 5 | 10.88 | 0.28 | 19.49 |
| | | 90 mg/mL of trehalose | 98.4 | 85.1 | 15 | 5 | 13.08 | 0.63 | 26.46 |
| | | 80 mg/mL of maltose | 96.0 | 86.6 | 18 | 8 | 11.61 | 0.47 | 18.78 |
| | | 20 mg/mL of glycerol | 98.3 | 85.3 | 23 | 10 | 11.03 | 0.1 | 18.18 |
| | | 220 mM of L-proline + 5 mM of L-methionine | 98.6 | 83.3 | 18 | 3 | 10.58 | 0.25 | 15.87 |
| | | 220 mM of L-proline + 1 mM of disodium EDTA | 98.5 | 83.3 | 18 | 3 | 11.74 | 0.18 | 16.54 |
| | | 100 mM of | 98.6 | 83.4 | 20 | 0 | 16.76 | 0.26 | 17.55 |
| | | L-arginine hydrochloride + 100 mM of L-proline | | | | | | | |
| | | 100 mM of sodium chloride | 98.4 | 85.5 | 25 | 5 | 20.11 | 0.48 | 18.56 |

After 28 days of high-temperature acceleration, the appearance visually inspected showed that except that the formula containing maltose was yellow in color, other formulas showed no significant difference in appearance.

SEC-HPLC detection results show that: except that the formula containing maltose showed relatively low monomer content, other formulas showed no significant difference in monomers detected (between 98.3% and 98.6%); WCX-HPLC detection results show that: except that the formula containing maltose showed change in peak pattern, other formulas showed no significant difference in principal component detected (between 83.3% and 85.5%). The detection results of insoluble particles show that: there was no significant difference in the presence of different stabilizers. DLS detection results: the average hydrodynamic diameter (Z. Av. Diameter) results show that the formula containing sodium chloride had a relatively larger hydrodynamic diameter, and the other formulas showed smaller average particle size; the distribution coefficient (PDI) results show that except that the formulas containing trehalose, maltose and sodium chloride had values >0.3, other formulas showed no significant difference and demonstrated good uniformity. The viscosity detection results show that: the viscosity of the formulas containing 50 mg/mL of sucrose, 50 mg/mL of mannitol and 90 mg/mL of trehalose was > 20 cp, and that of the other formulas was < 20 cp; the formula containing the antioxidant methionine was slightly less viscous than the formulas without methionine.

**Table 12. Detection results for different stabilizers after 28 days of acceleration at high temperature of 40±2 °C in the presence of the buffer L-histidine-L-histidine hydrochloride**

| PD-L1 monoclonal antibody Content | Other components | Stabilizer | SEC-HPLC monomer (%) | WCX-HPL C principal component (%) | Insoluble microparticle (per mL) | | DLS | |
|---|---|---|---|---|---|---|---|---|
| | | | | | 10µm | 25µm | Z.Av. Diameter (nm) | PDI |
| 200±12 mg/mL | 20 mM of L-histidine-L-hi stidine hydrochloride pH 6.4±0.1 0.02% polysorbate 20 | 220 mM of L-proline | 98.6 | 83.2 | 38 | 3 | 11.54 | 0.15 |
| | | 50 mg/mL of sucrose | 98.6 | 84.4 | 20 | 3 | 12.49 | 0.3 |
| | | 20 mg/mL of sucrose | 98.6 | 84.5 | 60 | 15 | 11.74 | 0.2 |
| | | 150 mM of L-arginine hydrochloride | 98.6 | 83.3 | 28 | 8 | 16.98 | 0.14 |
| | | 50 mM of L-arginine hydrochloride | 98.6 | 84.2 | 43 | 5 | 17.59 | 0.20 |
| | | 50 mg/mL of mannitol | 98.6 | 84.5 | 65 | 20 | 13.44 | 0.14 |
| | | 20 mg/mL of mannitol | 98.6 | 84.7 | 20 | 3 | 12.49 | 0.08 |
| | | 40 mg/mL of sorbitol | 98.7 | 84.6 | 10 | 3 | 11.7 | 0.21 |
| | | 90 mg/mL of trehalose | 98.7 | 84.6 | 30 | 3 | 14.9 | 0.41 |
| | | 80 mg/mL of maltose | 97.5 | 86.8 | 25 | 5 | 13.04 | 0.39 |
| | | 20 mg/mL of glycerol | 98.7 | 84.9 | 30 | 8 | 12.18 | 0.01 |
| | | 100 mM of L-arginine hydrochloride + 100 mM of L-proline | 98.9 | 83.4 | 48 | 5 | 17.36 | 0.07 |
| | | 100 mM of sodium chloride | 98.7 | 84.6 | 30 | 10 | 21.25 | 0.07 |

After 28 days of high-temperature acceleration, the appearance visually inspected showed that except that the formula containing maltose was yellow in color, other formulas showed no significant difference in appearance.

SEC-HPLC detection results show that: except that the formula containing maltose showed relatively low monomer content, other formulas showed no significant difference in monomers detected (between 98.6% and 98.9%); WCX-HPLC detection results show that: except that the formula containing maltose showed change in peak pattern, other formulas showed no significant difference in principal component detected (between 83.2% and 84.9%). The detection results of insoluble particles show that: there was no significant difference in the presence of different stabilizers. In the DLS detection, the Z. Av. Diameter results show that the formula containing sodium chloride had a relatively larger hydrodynamic diameter, and the other formulas showed smaller average particle size; the PDI results show that except that the formulas containing trehalose and maltose had PDI values >0.3, other formulas showed no significant difference and demonstrated good uniformity.

**Table 13. Detection results for different stabilizers after 28 days of acceleration at high temperature of 40±2 °C in the presence of the buffer citrate**

| PD-L1 monoclonal antibody Content | Other components | Stabilizer | Insoluble microparticle (per mL) | | DLS | |
|---|---|---|---|---|---|---|
| | | | 10µm | 25µm | Z.Av. Diameter(nm) | PDI |
| 200±12 mg/mL | 20 mM of citrate, pH 6.4±0.1 0.02% Tween 20 | 220 mM of L-proline | 15 | 5 | 21.27 | 0.45 |
| | | 50 mg/mL of sucrose | 28 | 3 | 23.59 | 0.32 |
| | | 150 mM of L-arginine hydrochloride | 25 | 8 | 18.3 | 0.08 |
| | | 50 mg/mL of mannitol | 20 | 10 | 25.78 | 0.18 |
| | | 100 mM of L-arginine hydrochloride + 100 mM of L-proline | 45 | 5 | 18.31 | 0.2 |

The detection results of insoluble particles show that: there was no significant difference in the presence of different stabilizers. DLS detection results show that: except that the Z. Av. Diameter values of the formulas containing L-arginine hydrochloride were not greatly different from the values obtained when other buffering systems were used, the other formulas containing other stabilizers had relatively large hydrodynamic diameters shown by Z. Av. Diameter values in the presence of the buffering system citrate compared with the values obtained in the presence of other two buffering systems, which is not beneficial to the structural stability of protein; the PDI values detected for formulas containing different stabilizers were different, and the formulas containing L-arginine hydrochloride and mannitol had relatively small PDI values and showed good uniformity.

### 3. Screening of surfactants

The stability of the PD-L1 monoclonal antibody in different formulas, in particular the influence of different surfactants on the stability of the protein, was examined. The purity difference of the protein was detected by SEC-HPLC and WCX-HPLC in the acceleration experiment (high temperature of 40±2 °C), insoluble particles were detected by shaking for 7 days at 150 rpm at 25±2 °C, and particles and uniformity difference in different formulas were detected by DLS. Specific formula composition and the detection results are shown in Table 14.

**Table 14. Examining and detection results for screening of different surfactants**

| Content of PD-L1 monoclonal antibody | No. | 20 mM of sodium acetate-ac etic acid | 220 of L-proline mM | Surfactant | Accelerating at 40±2 °C for 28 days | | Shaking at 150 rpm at 25±2 °C for 7 days | | | |
|---|---|---|---|---|---|---|---|---|---|---|
| | | | | | SEC-HPLC-monomer (%) | WCX-HPLC principal component (%) | Insoluble microparticle (per mL) | | DLS | |
| | | | | | | | 10µm | 25µm | Z.Av. Diameter(nm) | PDI |
| 200±12 mg/mL | 1 | - | - | - | 98.3 | 84.5 | 465 | 385 | 10.26 | 0.12 |
| | 2 | + | - | - | 98.1 | 85.0 | 980 | 775 | 9.83 | 0.31 |
| | 3 | - | + | - | 98.5 | 81.5 | 910 | 615 | 10.02 | 0.05 |
| | 4 | + | + | - | 98.6 | 83.7 | 625 | 300 | 10.02 | 0.36 |
| | 5 | + | + | +0.02% polysorbate 20 | 98.6 | 83.4 | 10 | 0 | 9.86 | 0.20 |
| | 6 | + | + | +0.02% polysorbate 80 | 98.5 | 83.2 | 0 | 0 | 10.24 | 0.18 |
| | 7 | + | + | +0.02% poloxamer 188 | 98.6 | 83.9 | 10 | 0 | 10.16 | 0.21 |

| | | | | | | | | | | |
|---|---|---|---|---|---|---|---|---|---|---|
| "-" represents not added to the formula, and "+" represents added to the formula | | | | | | | | | | |

After 28 days of acceleration at 40±2 °C, the SEC-HPLC detection results show that: different formulas showed no significant difference; the WCX-HPLC detection results show that: the formula only containing 220 mM of L-proline showed relatively significant reduction in principal component as compared to other formulas, and other formulas showed no significant difference in principal component.

After 7 days of shaking at 150 rpm at 25±2 °C, the insoluble particle detection results show that the number of insoluble particles of the formulas containing the surfactant was small, while that of other formulas without surfactant was relatively large. The DLS detection results show that adding different surfactants (polysorbate 20, polysorbate 80 or poloxamer 188) can improve PDI value, and the formulas containing different surfactants showed no significant difference in terms of this value.

### Example 4

Further optimization of the excipient and pH of the formula was performed based on the results of the previous examples, and the following formulation formulas were obtained according to Table 15:

**Table 15. Different compositions of formulation formulas in confirmation experiment**

| Formula | Content of PD-L1 monoclonal antibody (mg/mL) | Buffering system | Excipient | Surfactant | pH |
|---|---|---|---|---|---|
| 20 | 200±5 | 20 mM of sodium acetate-acetic acid | 150 mM of glycine | 0.02% polysorbate 20 | 6.1 |
| 21 | 200±5 | 20 mM of sodium acetate-acetic acid | 150 mM of glycine | 0.02% polysorbate 20 | 6.3 |
| 22 | 200±5 | 20 mM of sodium acetate-acetic acid | 150 mM of glycine | 0.02% polysorbate 20 | 6.5 |
| 23 | 200±5 | 20 mM of sodium acetate-acetic acid | 150 mM of glycine | 0.02% polysorbate 20 | 6.7 |
| 24 | 200±5 | 20 mM of sodium acetate-acetic acid | 220 mM of L-proline | 0.02% polysorbate 20 | 6.1 |
| 25 | 200±5 | 20 mM of sodium acetate-acetic acid | 220 mM of L-proline | 0.02% polysorbate 20 | 6.3 |
| 26 | 200±5 | 20 mM of sodium acetate-acetic acid | 220 mM of L-proline | 0.02% polysorbate 20 | 6.5 |
| 27 | 200±5 | 20 mM of sodium acetate-acetic acid | 220 mM of L-proline | 0.02% polysorbate 20 | 6.7 |
| 28 | 200±5 | 20 mM of sodium acetate-acetic acid | 150 mM of L-proline | 0.02% polysorbate 20 | 6.1 |
| 29 | 200±5 | 20 mM of sodium acetate-acetic acid | 150 mM of L-proline | 0.02% polysorbate 20 | 6.3 |
| 30 | 200±5 | 20 mM of sodium acetate-acetic acid | 150 mM of L-proline | 0.02% polysorbate 20 | 6.5 |
| 31 | 200±5 | 20 mM of sodium acetate-acetic acid | 150 mM of L-proline | 0.02% polysorbate 20 | 6.7 |

The formulation formulas in the Table 15 are screened and detected according to Table 16.

**Table 16. Detection items for confirmation of formulation formulas**

| Detection items | Duration of investigation (day) (temperature: 40±2 °C) | | |
|---|---|---|---|
| | 0 | 14 | 28 |
| SEC-HPLC | √ | √ | √ |
| WCX-HPLC | √ | √ | √ |
| Osmotic pressure | √ | -- | -- |

### 1) SEC-HPLC detection of main peak content

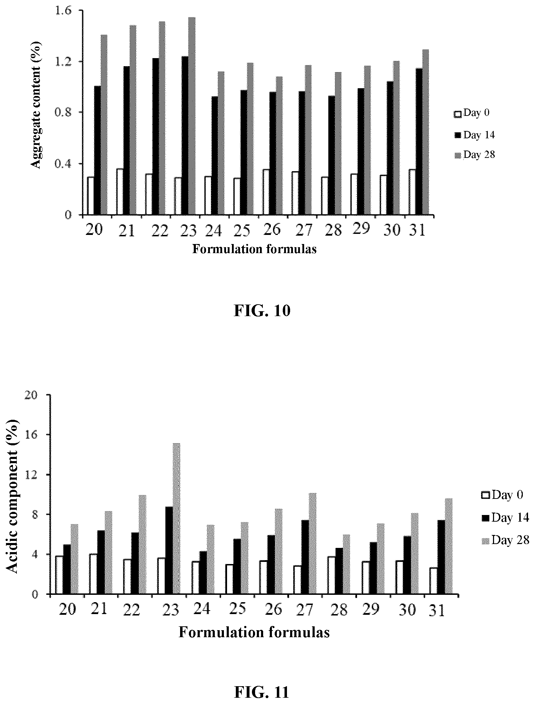

The results of the main peak content and the results of the aggregate content corresponding to formulas 20-31 as detected by SEC-HPLC are shown in FIG. 9 and FIG. 10, respectively.

Comparing the results of formulas 20-23, 24-27 and 28-31 separately, it can be seen that the difference in the effect of pH on stability is not significant when the pH value is within 6.1-6.7. Comparing the aggregate content of formulas 20-23 and 28-31 separately, it is found that the inclusion of L-proline correlates with further improvement in stability.

Comparing the results of formulas 24-27 and 28-31 separately, it is found that there was no significant difference in the effect of 220 mM of L-proline and 150 mM of L-proline on stability.

### 2) WCX-HPLC detection of acidic component content

The results of the acidic component content of the formulas 20-31 detected by WCX (after acceleration at high temperature of 40±2 °C) are shown in FIG. 11.

Comparing the acidic components of formulas 20-23, formulas 24-27, and formulas 28-31 separately, it can be seen that the acidic component content of formulas at pH of 6.1-6.7 tended to increase along with the increase of pH, but was all within an acceptable range.

Comparing the acidic components of formulas 20-23 and 28-31 separately, it is found that the inclusion of L-proline correlates with further improvement in stability.

Comparing the acidic components of formulas 24-27 and 28-31 separately, it is found that there was no significant difference in the effect of 220 mM of L-proline and 150 mM of L-proline on stability.

### 3) Detection of osmotic pressure

The results of the detection of osmotic pressure are shown in Table 17.

**Table 17. Detection results of osmotic pressure**

| Formula | Excipient | Osmotic pressure (mOsmoL/kg) |
|---|---|---|
| 20 | 150 mM of glycine | 306 |
| 24 | 220 mM of L-proline | 373 |
| 28 | 150 mM of L-proline | 296 |

Osmotic pressure that is less than 600 mOsmoL/kg is within safe limits for infusion according to INS (infusion nurses society) standards, and therefore the osmotic pressure of the formulas 20, 24 and 28 are within safe limits.

### Example 5

Through a series of experiments on protein content, pH range, screening of buffers and screening of stabilizers, a preferred combination (sodium acetate-acetic acid as the buffering system, L-proline as the stabilizer, and polysorbate 20 as the surfactant) is selected. The influence of the concentration of sodium acetate-acetic acid, the concentration of L-proline, the concentration of polysorbate 20, the content of PD-L1 monoclonal antibody protein and the pH on protein stability was further examined through DoE experimental design. The 5 factors were examined by using the classical two-level screening design of the JMP15.0 software, through which 18 combinations were obtained by designing 2 levels and 1 repetition of 1 central point. The experimental design is shown in Table 18 below.

**Table 18. Different compositions of formulation formulas in DOE experiment**

| Formula | Content of PD-L1 monoclonal antibody (mg/mL) | Concentration of sodium acetate (mM) | Concentratio n of L-proline (mM) | Concentration of polysorbate 20 (%) | pH |
|---|---|---|---|---|---|
| 1A | 50 | 35 | 50 | 0 | 5.9 |
| 2 A | 50 | 35 | 390 | 0 | 6.9 |
| 3 A | 220 | 5 | 390 | 0.04 | 5.9 |
| 4 A | 135 | 20 | 220 | 0.02 | 6.4 |
| 5 A | 220 | 5 | 390 | 0 | 6.9 |
| 6 A | 50 | 35 | 50 | 0.04 | 6.9 |
| 7 A | 220 | 35 | 390 | 0.04 | 6.9 |
| 8 A | 220 | 35 | 390 | 0 | 5.9 |
| 9 A | 220 | 35 | 50 | 0.04 | 5.9 |
| 10 A | 50 | 5 | 50 | 0.04 | 5.9 |
| 11 A | 50 | 5 | 50 | 0 | 6.9 |
| 12 A | 50 | 35 | 390 | 0.04 | 5.9 |
| 13 A | 50 | 5 | 390 | 0 | 5.9 |
| 14 A | 220 | 35 | 50 | 0 | 6.9 |
| 15 A | 220 | 5 | 50 | 0 | 5.9 |
| 16 A | 50 | 5 | 390 | 0.04 | 6.9 |
| 17 A | 135 | 20 | 220 | 0.02 | 6.4 |
| 18 A | 220 | 5 | 50 | 0.04 | 6.9 |

After being accelerated for 14 days and 28 days at high temperature (40±2 °C), the above formulas were detected by SEC-HPLC for monomer content and by WCX-HPLC for principal component content, and the results are summarized in Table 19.

**Table 19. Detection results of different formulas in DOE experimental design of formulation composition**

| No. | SEC-HPLC monomer (%) | | | WCX-HPLC principal component (%) | | |
|---|---|---|---|---|---|---|
| | Day 0 | Day 14 | Day 28 | Day 0 | Day 14 | Day 28 |
| 1A | 99.6 | 99.5 | 99.1 | 92.4 | 89.6 | 86.5 |
| 2A | 99.4 | 99.0 | 98.8 | 92.1 | 84.3 | 77.3 |
| 3A | 99.5 | 98.9 | 98.6 | 92.0 | 88.9 | 85.1 |
| 4A | 99.6 | 99.0 | 98.7 | 92.2 | 87.8 | 83.4 |
| 5A | 99.4 | 98.5 | 98.1 | 91.8 | 83.1 | 75.7 |
| 6A | 99.3 | 98.6 | 98.6 | 92.2 | 86.0 | 80.8 |
| 7A | 99.3 | 98.5 | 98.0 | 91.9 | 84.2 | 77.8 |
| 8A | 99.5 | 98.8 | 98.5 | 92.2 | 88.4 | 85.0 |
| 9A | 99.5 | 98.8 | 98.3 | 92.3 | 89.5 | 85.9 |
| 10A | 99.6 | 99.3 | 99.1 | 92.5 | 89.7 | 87.2 |
| 11A | 99.6 | 99.1 | 98.7 | 92.6 | 85.9 | 80.2 |
| 12A | 99.3 | 99.3 | 99.2 | 93.6 | 89.2 | 85.8 |
| 13A | 99.6 | 99.4 | 99.3 | 92.7 | 89.1 | 85.9 |
| 14A | 99.3 | 98.6 | 97.8 | 92.8 | 86.5 | 81.5 |
| 15A | 99.5 | 98.7 | 98.3 | 92.2 | 89.1 | 86.1 |
| 16A | 99.5 | 99.1 | 98.9 | 92.6 | 82.5 | 74.2 |
| 17A | 99.6 | 99.0 | 98.8 | 93.4 | 88.1 | 83.1 |
| 18A | 99.3 | 98.3 | 97.7 | 91.8 | 85.9 | 81.1 |

The results show that: the concentration of the buffering system sodium acetate (5-35 mM), the concentration of the stabilizer L-proline (50-390 mM) and the concentration of polysorbate 20 (0-0.04%) had no significant influence on the SEC-HPLC monomer and WCX-HPLC principal component in protein detection, and the change was small within the ranges listed. The protein content and pH had influence on the SEC-HPLC monomer and WCX-HPLC principal component in protein detection, and the change was large within the ranges listed. After 28 days of high temperature, the SEC-HPLC monomer purity was no less than 95% when the protein concentration was 50-220 mg/mL, and the WCX-HPLC principal component was no less than 74% when the pH was between 5.9 and 6.9, which were both in an acceptable range and therefore the change was determined to be acceptable.

### Example 6

Accelerated stability study was performed on the preferred formula. The formula composition of the sample was as follows: 200±20 mg/mL of PD-L1 monoclonal antibody protein, 20 mM of sodium acetate-acetic acid, 220 mM of L-proline, and 0.02% polysorbate 20, pH 6.4±0.1. The sample aliquots were placed in vials, stored at 25±2 °C for 9 months, and sampled at the end of months 0, 3, 6 and 9. Based on the key examination items for stability detection, the purity of the sample monomer (SEC-HPLC), the purity of the principal component in the charge distribution (WCX-HPLC), the reduced and non-reduced capillary gel electrophoresis (CE-SDS), the protein content, etc., were examined, and the results are shown in Table 20.

**Table 20. Data of accelerated (25±2 °C, 9 months) stability study**

| Detection indexes | Examination condition: 25±2 °C (month) | | | |
|---|---|---|---|---|
| | 0 | 3 | 6 | 9 |
| SEC-HPLC monomer (%) | 99.6 | 99.2 | 99.1 | 98.8 |
| WCX-HPLC principal component (%) | 92.5 | 86.5 | 83.8 | 78.0 |
| CE-SDS reduced purity (%) | 98.0 | 96.0 | 94.9 | 93.8 |
| CE-SDS non-reduced purity (%) | 98.5 | 96.5 | 94.4 | 92.1 |
| Protein content (mg/mL) | 184.1 | 187.1 | 186.8 | 187.2 |

The results show that when the sample was stored at 25±2 °C for 9 months, all indexes of the sample were within the acceptable range compared with the results on day 0. This suggests that the formulation formula of the present invention can still maintain its stability after being stored at room temperature for 9 months.

### Example 7

Long-term stability study was performed on the preferred formula. The formula composition of the sample was as follows: 200±20 mg/mL of PD-L1 monoclonal antibody protein, 20 mM of sodium acetate-acetic acid, 220 mM of L-proline, and 0.02% polysorbate 20, pH 6.4±0.1. The sample aliquots were placed in vials, stored at 5±3°C for 18 months, and sampled at the end of months 0, 6, 12 and 18. Based on the key examination items for stability detection, the purity of the sample monomer (SEC-HPLC), the purity of the principal component in the charge distribution (WCX-HPLC), the reduced and non-reduced capillary gel electrophoresis (CE-SDS), the protein content, etc., were examined, and the results are shown in Table 21.

**Table 21. Data of long-term (5±3°C, 18 months) stability study**

| Detection indexes | Examination condition: 5±3°C (month) | | | |
|---|---|---|---|---|
| | 0 | 6 | 12 | 18 |
| SEC-HPLC monomer (%) | 99.6 | 99.4 | 99.4 | 99.2 |
| WCX-HPLC principal component (%) | 92.0 | 90.2 | 89.2 | 89.7 |
| CE-SDS reduced purity (%) | 97.9 | 97.8 | 97.3 | 96.1 |
| CE-SDS non-reduced purity (%) | 98.6 | 98.2 | 97.8 | 99.9 |
| Protein content (mg/mL) | 190.7 | 191.6 | 190.2 | 191.5 |

The results show that when the sample was stored at 5±3°C for 18 months, related indexes of the sample detection were all within the acceptable range compared with the results on day 0. This suggests that the formulation formula of the present invention can still maintain its stability after being stored at 5±3 °C for 18 months.

The foregoing detailed description is provided by way of illustration and example, and is not intended to limit the scope of the appended claims. Various variants of the embodiments currently listed in the present application will be apparent to those of ordinary skill in the art, and are intended to be within the scope of the appended claims and equivalents thereof.

## Claims

1. A composition, comprising an antigen-binding fragment and an excipient, wherein the antigen-binding fragment comprises an immunoglobulin single variable domain capable of specifically binding to PD-L1 and comprising CDR1-3, wherein the CDR3 comprises an amino acid sequence set forth in SEQ ID NO: 47, and
the excipient is selected from one or more of the group consisting of: proline, mannitol, sucrose, glycine and L-arginine hydrochloride, or
the excipient is selected from one or more of the group consisting of: proline, sucrose, mannitol, sorbitol and glycerol.

2. The composition according to claim 1, wherein the CDR2 comprises an amino acid sequence set forth in SEQ ID NO: 46.

3. The composition according to any one of claims 1-2, wherein the CDR1 comprises an amino acid sequence set forth in SEQ ID NO: 45 or 48.

4. The composition according to any one of claims 1-3, wherein the immunoglobulin single variable domain comprises an amino acid sequence set forth in any one of SEQ ID NOs: 1-6.

5. The composition according to any one of claims 1-4, wherein the PD-L1 is human PD-L1.

6. The composition according to any one of claims 1-5, wherein the antigen-binding fragment comprises an Fc region of an immunoglobulin.

7. The composition according to claim 6, wherein the N-terminus of the Fc region of the immunoglobulin is linked directly or indirectly to the C-terminus of the immunoglobulin single variable domain.

8. The composition according to any one of claims 6-7, wherein the N-terminus of the Fc region of the immunoglobulin is linked to the C-terminus of the immunoglobulin single variable domain by a linker.

9. The composition according to claim 8, wherein the linker comprises an amino acid sequence set forth in any one of SEQ ID NOs: 42-44.

10. The composition according to any one of claims 6-9, wherein the Fc region of the immunoglobulin comprises an Fc region derived from an immunoglobulin selected from the group consisting of: IgG1, IgG2, IgG3 and IgG4.

11. The composition according to any one of claims 6-10, wherein the Fc region of the immunoglobulin has no ADCC activity; and/or, the Fc region of the immunoglobulin has no CDC activity.

12. The composition according to any one of claims 6-11, wherein the Fc region of the immunoglobulin is derived from a human being.

13. The composition according to any one of claims 6-12, wherein the Fc region of the immunoglobulin is derived from human IgG.

14. The composition according to any one of claims 6-13, wherein the Fc region of the immunoglobulin comprises an amino acid sequence set forth in any one of SEQ ID NOs: 7-9.

15. The composition according to any one of claims 1-14, wherein the antigen-binding fragment comprises an amino acid sequence set forth in any one of SEQ ID NOs: 14-31.

16. The composition according to any one of claims 1-15, wherein the antigen-binding fragment is at a concentration of about 1 mg/mL to about 220 mg/mL.

17. The composition according to any one of claims 1-16, wherein the antigen-binding fragment is at a concentration of about 20 mg/mL to about 220 mg/mL.

18. The composition according to any one of claims 1-17, wherein the antigen-binding fragment is at a concentration of about 50 mg/mL to about 200 mg/mL.

19. The composition according to any one of claims 1-17, wherein the antigen-binding fragment is at a concentration of about 180 mg/mL to about 220 mg/mL.

20. The composition according to any one of claims 1-15, wherein the antigen-binding fragment is at a concentration of about 100 mg/mL to about 280 mg/mL.

21. The composition according to any one of claims 1-15, wherein the antigen-binding fragment is at a concentration of about 150 mg/mL to about 250 mg/mL.

22. The composition according to any one of claims 1-21, wherein the excipient is at a concentration of about 50 mM to about 500 mM.

23. The composition according to any one of claims 1-22, wherein the excipient comprises proline at a concentration of about 50 mM to about 390 mM.

24. The composition according to any one of claims 1-23, wherein the excipient comprises proline at a concentration of about 150 mM to about 250 mM.

25. The composition according to any one of claims 1-24, wherein the excipient comprises proline at a concentration of about 150 mM to about 220 mM.

26. The composition according to any one of claims 1-25, wherein the excipient comprises L-proline at a concentration of about 150 mM to about 220 mM.

27. The composition according to any one of claims 1-26, wherein the excipient comprises L-proline at a concentration of about 165 mM to about 275 mM.

28. The composition according to any one of claims 1-27, wherein the excipient comprises mannitol at a concentration of about 50 mM to about 500 mM.

29. The composition according to any one of claims 1-28, wherein the excipient comprises sucrose at a concentration of about 50 mM to about 500 mM.

30. The composition according to any one of claims 1-29, wherein the excipient comprises sucrose at a concentration of about 90 mM to about 500 mM.

31. The composition according to any one of claims 1-30, wherein the excipient comprises glycine at a concentration of about 50 mM to about 500 mM.

32. The composition according to any one of claims 1-31, wherein the excipient comprises glycine at a concentration of about 150 mM to about 250 mM.

33. The composition according to any one of claims 1-32, wherein the excipient comprises L-arginine hydrochloride at a concentration of about 50 mM to about 500 mM.

34. The composition according to any one of claims 1-33, wherein the excipient comprises L-arginine hydrochloride at a concentration of about 150 mM to about 500 mM.

35. The composition according to any one of claims 1-34, wherein the excipient comprises sorbitol at a concentration of about 50 mM to about 500 mM.

36. The composition according to any one of claims 1-35, wherein the excipient comprises sorbitol at a concentration of about 100 mM to about 300 mM.

37. The composition according to any one of claims 1-36, wherein the excipient comprises glycerol at a concentration of 50 mM to about 500 mM.

38. The composition according to any one of claims 1-37, wherein the excipient comprises glycerol at a concentration of 100 mM to about 300 mM.

39. The composition according to any one of claims 1-38, wherein the composition has a pH of about 5.0 to about 7.5.

40. The composition according to any one of claims 1-39, wherein the composition has a pH of about 5.9 to about 6.9.

41. The composition according to any one of claims 1-40, wherein the composition has a pH of about 6.3 to about 6.5.

42. The composition according to any one of claims 1-41, wherein the composition has a pH of about 6.4.

43. The composition according to any one of claims 1-42, comprising a buffering component, wherein the buffering component is selected from one or more of the group consisting of sodium acetate-acetic acid, histidine-acetic acid, L-histidine-L-histidine hydrochloride, sodium phosphate and citric acid-sodium hydroxide.

44. The composition according to claim 43, wherein the buffering component is at a concentration of about 5 mM to about 50 mM.

45. The composition according to any one of claims 43-44, wherein the buffering component comprises sodium acetate-acetic acid at a concentration of about 5 mM to about 35 mM.

46. The composition according to any one of claims 43-45, wherein the buffering component comprises sodium acetate-acetic acid at a concentration of about 10 mM to about 30 mM.

47. The composition according to any one of claims 43-46, wherein the buffering component comprises sodium acetate-acetic acid at a concentration of about 20 mM.

48. The composition according to any one of claims 1-47, comprising a surfactant, wherein the surfactant is selected from one or more of the group consisting of: polysorbate 20, polysorbate 80 and poloxamer 188.

49. The composition according to claim 48, wherein the surfactant is at a concentration of about 0 mg/mL to about 0.8 mg/mL.

50. The composition according to any one of claims 48-49, wherein the surfactant comprises polysorbate 20 at a concentration of about 0 mg/mL to about 0.4 mg/mL.

51. The composition according to any one of claims 48-50, wherein the surfactant comprises polysorbate 20 at a concentration of about 0.2 mg/mL to about 0.4 mg/mL.

52. The composition according to any one of claims 48-50, wherein the surfactant comprises polysorbate 20 at a concentration of about 0.1 mg/mL to about 0.3 mg/mL.

53. The composition according to any one of claims 48-52, wherein the surfactant comprises polysorbate 20 at a concentration of about 0.2 mg/mL.

54. The composition according to any one of claims 1-53, comprising:
1) about 20 mg/mL to about 220 mg/mL of the antigen-binding fragment according to any one of claims 1-15;
2) about 5 mM to about 35 mM of sodium acetate-acetic acid;
3) about 50 mM to about 390 mM of proline; and
4) about 0 mg/mL to about 0.4 mg/mL of polysorbate 20;
wherein the composition has a pH of about 5.9 to about 6.9.

55. The composition according to any one of claims 1-54, comprising:
1) about 50 mg/mL to about 220 mg/mL of the antigen-binding fragment according to any one of claims 1-15;
2) about 10 mM to about 30 mM of sodium acetate-acetic acid;
3) about 150 mM to about 250 mM of proline; and
4) about 0.2 mg/mL to about 0.4 mg/mL of polysorbate 20;
wherein the composition has a pH of about 6.3 to about 6.5.

56. The composition according to any one of claims 1-55, comprising:
1) about 200 mg/mL of the antigen-binding fragment according to any one of claims 1-15;
2) about 20 mM of sodium acetate-acetic acid;
3) about 220 mM of L-proline; and
4) about 0.2 mg/mL polysorbate 20;
wherein the composition has a pH of about 6.4.

57. The composition according to any one of claims 1-56, wherein the composition is used for inj ection.

58. The composition according to any one of claims 1-57, wherein the composition is used for subcutaneous injection.

59. The composition according to any one of claims 1-58, wherein the composition is a formulation.

60. The composition according to any one of claims 1-59, wherein the composition is a liquid formulation.

61. A kit, comprising the composition according to any one of claims 1-60 and a container holding the composition according to any one of claims 1-60.

62. The kit according to claim 61, wherein the container comprises a glass vial.

63. The kit according to any one of claims 61-62, wherein the composition in the container has a volume of about 0.5 mL to about 5.0 mL.

64. The kit according to any one of claims 61-63, wherein the composition in the container has a volume of about 0.5 mL to about 1.5 mL.

65. Use of the composition according to any one of claims 1-60 and/or the kit according to any one of claims 61-64 in the preparation of a medicament for preventing, alleviating and/or treating a tumor.

66. The use according to claim 65, wherein the tumor comprises a PD-L1 positive tumor.

67. The use according to any one of claims 65-66, wherein the tumor comprises a malignant solid tumor.

68. Use of the composition according to any one of claims 1-60 and/or the kit according to any one of claims 61-64 in the preparation of a medicament for preventing, alleviating and/or treating an infectious disease.

69. The use according to claim 68, wherein the infectious disease comprises a disease caused by a viral infection, a bacterial infection, a fungal infection and/or a parasitic infection.
